# EUROPÄISCHE PATENTSCHRIFT

(11) **EP 3 372 580 B1**
(45) Veröffentlichungstag und Bekanntmachung des Hinweises auf die Patenterteilung: **22.07.2020**
(21) Anmeldenummer: 18162778.7
(22) Anmeldetag: 20.01.2012
(51) Int. Cl.: C07D 207/36, C07D 491/113, C07D 491/107, C07C 255/46, C07D 317/72, C07C 235/74, C07D 209/54, C07D 307/22

(54) **VERFAHREN ZUR HERSTELLUNG VON 1-H-PYRROLIDIN-2,4-DION-DERIVATEN**
METHOD FOR THE PREPARATION OF 1-H-PYRROLIDIN-2,4-DIONE-DERIVATIVES
PROCÉDÉ DE PRODUCTION DE DÉRIVÉS DE LA 1-H-PYRROLIDINE-2,4-DIONE

(30) Priorität: 25.01.2011 EP 11152069; 25.01.2011 US 201161435910 P
(43) Veröffentlichungstag der Anmeldung: 12.09.2018
(62) Teilanmeldung aus: 12700832.4
(73) Patentinhaber: Bayer CropScience Aktiengesellschaft, 40789 Monheim (DE)
(72) Erfinder: FISCHER, Reiner, 40789 Monheim (DE); HIMMLER, Thomas, 51519 Odenthal (DE)
(74) Vertreter: BIP Patents

(56) Entgegenhaltungen:
- DE-A1- 4 409 044
- DE-A1- 19 515 690
- WAYLAND E. NOLAND, RICHARD J. SUNDBERG: "Structure of the 2:2 Condensation Product of Nitromethane and Cyclohexanone", JOURNAL OF ORGANIC CHEMISTRY, Bd. 28, Nr. 11, 1963, Seiten 3150-3165, XP002632565, DOI: 10.1021/jo01046a051

## Beschreibung

Die vorliegende Erfindung betrifft ein neues Verfahren zur Herstellung von 1-H-Pyrrolidin-2,4-dion-Derivaten und neue Zwischenprodukte sowie ein Verfahren zu ihrer Herstellung.

1-H-Pyrrolidin-2,4-dion-Derivate mit akarizider, insektizider, fungizider und herbizider Wirkung sind bekannt:
EP-A-456 063, EP-A-521 334, EP-A-596 298, EP-A-613 884, EP-A-613 885, WO 95/01 971, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/24437, WO 99/43649, WO 99/48869, WO 99/55673, WO 01/17972, WO 01/23354, WO 01/74770, WO 03/013249, WO 03/062244, WO 2004/007448, WO 2004/024 688, WO 04/065366, WO 04/080962, WO 04/111042, WO 05/044791, WO 05/044796, WO 05/048710, WO 05/049569, WO 05/066125, WO 05/092897, WO 06/000355, WO 06/029799, WO 06/056281, WO 06/056282, WO 06/089633, WO 07/048545, DEA 102 00505 9892, WO 07/073856, WO 07/096058, WO 07/121868, WO 07/140881, WO 08/067873, WO 08/067910, WO 08/067911, WO 08/138551, WO 09/015801, WO 09/039975, WO 09/049851, WO 09/115262, WO 10/052161, WO 10/102758, WO 10/063378, WO 10/063670, WO 10/102758, WO 2011/098443, WO 2011/098440, WO 11/067135, WO 11/067240, EP-Anmeldenummer 11154805.3. Außerdem sind ketalsubstituierte 1-H-Arylpyrrolidin-2,4-dione aus WO 99/16748 bekannt. Mit pharmazeutischer Wirkung sind bekannt WO 2011/098433, DE-A-102010008642, DE-A-102010008643 und DE-Anmeldenummer 102010008640.

Auch sind biphenylsubstituierte 1H-Pyrrolidin-dion Derivate mit fungizider Wirkung bekannt (WO 03/059065).

Es handelt sich dabei um Verbindungen der Formel (I) 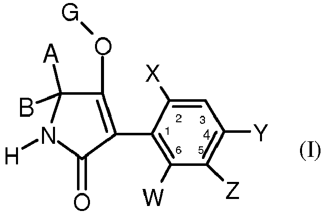 in welcher
W für Wasserstoff, Halogen, Alkyl, Alkenyl, Alkinyl, gegebenenfalls substituiertes Cycloalkyl, Alkoxy, Halogenalkyl oder Halogenalkoxy steht,
X für Wasserstoff, Halogen, Alkyl, Alkenyl, Alkinyl, gegebenenfalls substituiertes Cycloalkyl, Alkoxy, Halogenalkyl, Halogenalkoxy oder Cyano steht,
Y und Z unabhängig voneinander für Wasserstoff, Alkyl, Alkenyl, Alkinyl, Halogen, Cyano, gegebenenfalls substituiertes Cycloalkyl, Alkoxy, Halogenalkyl, Halogenalkoxy oder jeweils gegebenenfalls substituiertes Aryl oder Hetaryl stehen,
A für Wasserstoff, für jeweils gegebenenfalls durch Halogen substituiertes Alkyl, Alkenyl, Alkoxyalkyl, Alkylthioalkyl, gesättigtes oder ungesättigtes, gegebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
B für Wasserstoff, Alkyl oder Alkoxyalkyl steht, oder
A und B gemeinsam mit dem Kohlenstoffatom an das sie gebunden sind für einen gesättigten oder ungesättigten, gegebenenfalls mindestens ein Heteroatom enthaltenden unsubstituierten oder substituierten Cyclus stehen,
G für Wasserstoff (a) oder für eine der Gruppen 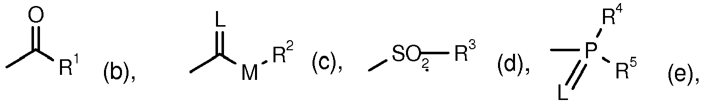 E (f) oder 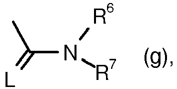 steht,
   worin
   E für ein Metallion oder ein Ammoniumion steht,
   L für Sauerstoff oder Schwefel steht,
   M für Sauerstoff oder Schwefel steht,
   R¹ für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
   R² für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloalkyl, Phenyl oder Benzyl steht,
   R³, R⁴ und R⁵ unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,
   R⁶ und R⁷ unabhängig voneinander für Wasserstoff, für jeweils gegebenenfalls durch Halogen oder Cyano substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden.

Die Verbindungen der Formel (I) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der verschiedenen Bedeutungen (a), (b), (c), (d), (e), (f) und (g) der Gruppe G ergeben sich folgende hauptsächliche Strukturen (I-a) bis (I-g), 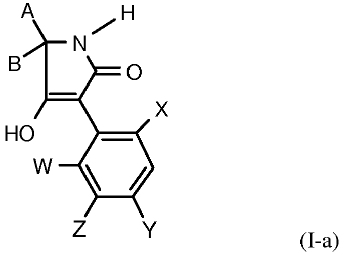 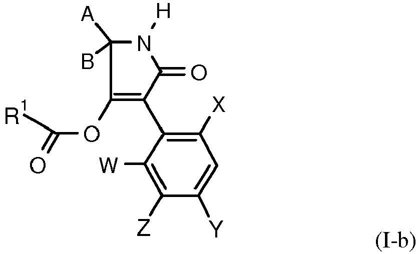 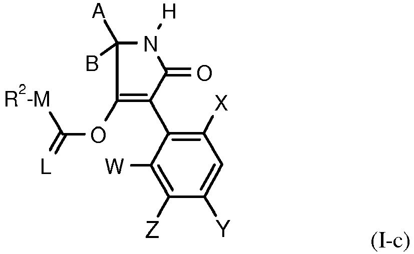 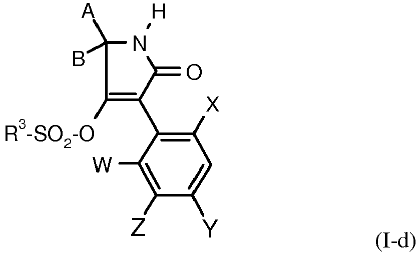 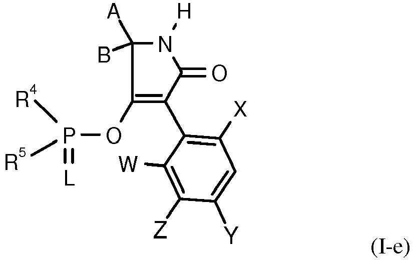 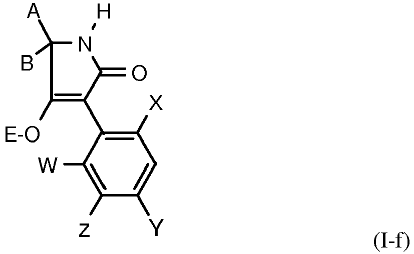 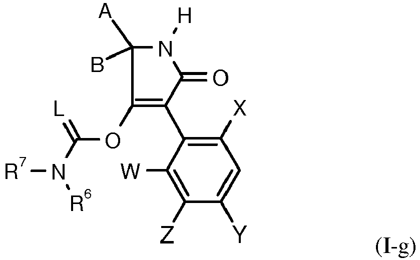 worin
A, B, E, L, M, W, X, Y, Z, R¹, R², R³, R⁴, R⁵, R⁶ und R⁷ die oben angegebenen Bedeutungen besitzen.

Weiterhin ist bereits bekannt, dass man die Verbindungen der Formel (I) nach den im Folgenden beschriebenen Verfahren erhält:
(A*) Man erhält Verbindungen der Formel (I-a) 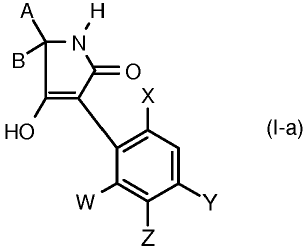 in welcher
A, B, W, X, Y und Z die oben angegebenen Bedeutungen haben,
wenn man
Verbindungen der Formel (II) 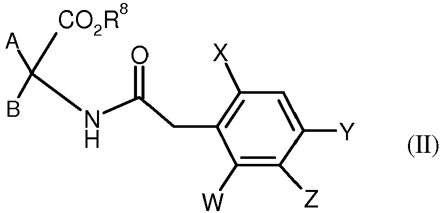 in welcher
A, B, W, X, Y und Z die oben angegebenen Bedeutungen haben,
und
R⁸ für Alkyl steht,
in Gegenwart eines Verdünnungsmittels und in Gegenwart einer Base intramolekular kondensiert.
Außerdem ist bekannt
- (B*): dass man die Verbindungen der oben gezeigten Formeln (I-b), in welchen R¹, A, B, W, X, Y und Z die oben angegebenen Bedeutungen haben, erhält, wenn man Verbindungen der oben gezeigten Formeln (I-a), in welchen A, B, W, X, Y und Z die oben angegebenen Bedeutungen haben, jeweils
- α): mit Verbindungen der Formel (III) 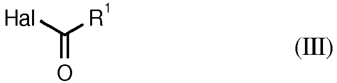
in welcher
- R¹: die oben angegebene Bedeutung hat und
- Hal: für Halogen (insbesondere Chlor oder Brom) steht
oder
- β): mit Carbonsäureanhydriden der Formel (IV)

R¹-CO-O-CO-R¹ (IV)

in welcher
R¹ die oben angegebene Bedeutung hat, gegebenenfalls in Gegenwart eines Verdünnungsmittels und gegebenenfalls in Gegenwart eines Säurebindemittels umsetzt;
- (C*): dass man die Verbindungen der oben gezeigten Formeln (I-c), in welchen R², A, B, M, W, X, Y und Z die oben angegebenen Bedeutungen haben und L für Sauerstoff steht, erhält, wenn man Verbindungen der oben gezeigten Formeln (I-a), in welchen A, B, W, X, Y und Z die oben angegebenen Bedeutungen haben, jeweils
mit Chlorameisensäureestern oder Chlorameisensäurethioestern der Formel (V)

R²-M-CO-Cl (V)

in welcher
R² und M die oben angegebenen Bedeutungen haben,
gegebenenfalls in Gegenwart eines Verdünnungsmittels und gegebenenfalls in Gegenwart eines Säurebindemittels umsetzt;
- (D*): dass man Verbindungen der oben gezeigten Formeln (I-f), in welchen E, A, B, W, X, Y und Z die oben angegebenen Bedeutungen haben, erhält, wenn man Verbindungen der Formeln (I-a), in welchen A, B, W, X, Y und Z die oben angegebenen Bedeutungen haben, jeweils
mit Metallverbindungen oder Aminen der Formeln (XIII) oder (XIV) 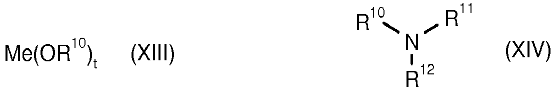 in welchen
- Me: für ein ein- oder zweiwertiges Metall (bevorzugt ein Alkali- oder Erdalkalimetall wie Lithium, Natrium, Kalium, Magnesium oder Calcium),
- t: für die Zahl 1 oder 2 und
- R¹⁰, R¹¹, R¹²: unabhängig voneinander für Wasserstoff oder Alkyl (bevorzugt C₁-C₈-Alkyl) stehen,
gegebenenfalls in Gegenwart eines Verdünnungsmittels umsetzt.

Die Verbindungen sind durch die Formel (I) allgemein definiert. Bevorzugte Substituenten bzw. Bereiche der in der oben und nachstehend erwähnten Formeln aufgeführten Reste werden im Folgenden erläutert:
- W: steht bevorzugt für Wasserstoff, Chlor, Brom, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₂-Halogenalkyl oder C₁-C₂-Halogenalkoxy,
- X: steht bevorzugt für Wasserstoff, Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₄-Halogenalkyl, C₁-C₄-Halogenalkoxy oder Cyano,
- Y und Z: stehen bevorzugt unabhängig voneinander für Wasserstoff, Cyano, Fluor, Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₆-Alkoxy, C₁-C₄-Halogenalkyl, C₁-C₄-Halogenalkoxy, oder für einen der (Het)-arylreste, 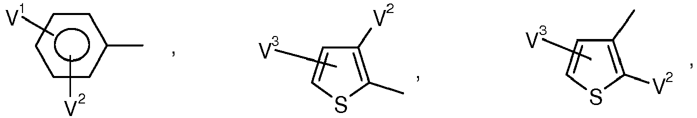 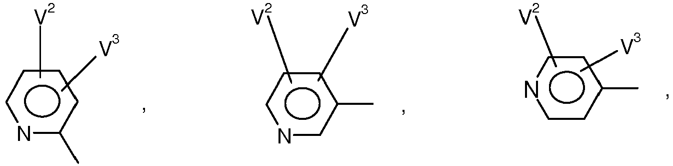 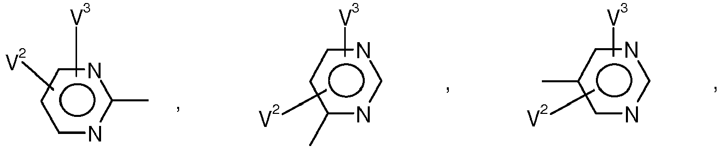 wobei im Falle von (Het)-aryl nur einer der Reste Y oder Z für (Het)-aryl stehen darf,
- V¹: steht bevorzugt für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro, Cyano oder gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl,
- V² und V³: stehen bevorzugt unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogenalkoxy,
- A: steht bevorzugt für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebenenfalls einfach bis zweifach durch C₁-C₂-Alkyl oder C₁-C₂-Alkoxy substituiertes und gegebenenfalls durch ein Sauerstoffatom unterbrochenes C₃-C₆-Cycloalkyl oder jeweils gegebenenfalls einfach bis zweifach durch Fluor, Chlor, Brom, C₁-C₄-Alkyl, C₁-C₂-Halogenalkyl, C₁-C₄-Alkoxy, C₁-C₂-Halogenalkoxy, Cyano oder Nitro substituiertes Phenyl, Pyridyl oder Benzyl,
- B: steht bevorzugt für Wasserstoff, C₁-C₄-Alkyl oder C₁-C₂-Alkoxyl-C₁-C₂-alkyl oder
- A, B: und das Kohlenstoffatom an das sie gebunden sind, stehen bevorzugt für gesättigtes oder ungesättigtes C₃-C₇-Cycloalkyl, worin gegebenenfalls ein Ringglied durch Stickstoff, Sauerstoff oder Schwefel ersetzt ist und welches gegebenenfalls einfach bis zweifach durch Halogen, C₁-C₆-Alkyl, C₁-C₄-Alkoxy-C₁-C₂-alkyl, Trifluormethyl, C₁-C₆-Alkoxy, C₃-C₆-Alkenyloxy, Trifluorethoxy, C₁-C₃-Alkoxy-C₁-C₃-alkoxy oder C₃-C₆-Cycloalkylmethoxy substituiert ist, wobei die zuvor genannten Reste (ausgenommen Halogen und Trifluormethyl) auch als N-Substituenten in Frage kommen, oder
- A, B: und das Kohlenstoffatom, an das sie gebunden sind, stehen bevorzugt für C₅-C₆-Cycloalkyl, welches durch eine gegebenenfalls mit ein oder zwei nicht direkt benachbarte Sauerstoff- oder Schwefelatome enthaltende gegebenenfalls durch Methyl oder Ethyl substituierte Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet, oder
- A, B: und das Kohlenstoffatom, an das sie gebunden sind, stehen bevorzugt für C₃-C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₂-Alkyl oder C₁-C₂-Alkoxy substituiertes C₂-C₄-Alkandiyl, C₂-C₄-Alkendiyl oder Butadiendiyl stehen,
- G: steht bevorzugt für Wasserstoff (a) oder für eine der Gruppen 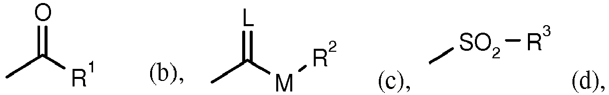 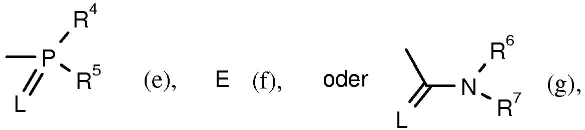
in welchen
E für ein Metallion oder ein Ammoniumion steht,
L für Sauerstoff oder Schwefel steht und
M für Sauerstoff oder Schwefel steht,
R¹ steht bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
   für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
   für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
   für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
   für gegebenenfalls einfach bis zweifach durch Fluor, Chlor, Brom oder C₁-C₄-Alkyl substituiertes Phenoxy-C₁-C₅-alkyl oder
   für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl,
R² steht bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
   für gegebenenfalls einfach bis zweifach durch Fluor, Chlor, C₁-C₄-Alkyl oder C₁-C₄-Alkoxy substituiertes C₃-C₇-Cycloalkyl oder
   für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl,
R³ steht bevorzugt für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl,
R⁴ und R⁵ stehen unabhängig voneinander bevorzugt für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, oder C₁-C₃-Halogenalkyl substituiertes Phenyl, Phenoxy oder Phenylthio,
R⁶ und R⁷ stehen unabhängig voneinander bevorzugt für Wasserstoff, für jeweils gegebenenfalls einfach bis dreifach durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls einfach bis dreifach durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.

In den als bevorzugt genannten Restedefinitionen steht Halogen für Fluor, Chlor und Brom, insbesondere für Fluor und Chlor.
- W: steht besonders bevorzugt für Wasserstoff, Chlor, Brom, Methyl, Ethyl, Methoxy, Ethoxy oder Trifluormethyl,
- X: steht besonders bevorzugt für Wasserstoff, Chlor, Brom, Jod, Methyl, Ethyl, Propyl, Cyclopropyl, Methoxy, Ethoxy, Trifluormethyl oder Trifluormethoxy,
- Y und Z: stehen besonders bevorzugt unabhängig voneinander für Wasserstoff, Fluor, Chlor, Brom, Iod, Methyl, Ethyl, Cyclopropyl, Methoxy, Trifluormethyl, Trifluormethoxy oder einen Phenylrest, 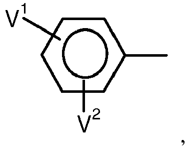
wobei im Falle von Phenyl nur einer der Reste Y oder Z für Phenyl stehen darf,
- V¹: steht besonders bevorzugt für Wasserstoff, Fluor oder Chlor,
- V²: steht besonders bevorzugt für Wasserstoff, Fluor, Chlor, Methyl, Ethyl, n-Propyl, iso-Propyl, Methoxy, Ethoxy oder Trifluormethyl,
- A: steht besonders bevorzugt für Wasserstoff, jeweils gegebenenfalls einfach bis dreifach durch Fluor substituiertes C₁-C₄-Alkyl oder C₁-C₂-Alkoxy-C₁-C₂-alkyl, für Cyclopropyl, Cyclopentyl oder Cyclohexyl,
- B: steht besonders bevorzugt für Wasserstoff, Methyl oder Ethyl oder
- A, B: und das Kohlenstoffatom an das sie gebunden sind, stehen besonders bevorzugt für gesättigtes C₅-C₆-Cycloalkyl, in welchem gegebenenfalls ein Ringglied durch Stickstoff, Sauerstoff oder Schwefel ersetzt ist und welches gegebenenfalls einfach oder zweifach durch Fluor, Chlor, Methyl, Ethyl, Methoxymethyl, Ethoxymethyl, Methoxyethyl, Ethoxyethyl, Trifluormethyl, Methoxy, Ethoxy, Propoxy, Butoxy, Methoxyethoxy, Ethoxyethoxy, Allyloxy, Trifluorethoxy oder Cyclopropylmethoxy substituiert ist, wobei die zuvor genannten Reste (ausgenommen Fluor, Chlor, Trifluormethyl) auch als N-Substituenten in Frage kommen,
- A, B: und das Kohlenstoffatom, an das sie gebunden sind, stehen besonders bevorzugt für C₆-Cycloalkyl, welches gegebenenfalls durch eine gegebenenfalls durch eine mit einem Sauerstoffatom unterbrochene Alkylidendiyl-Gruppe oder durch eine mit zwei nicht direkt benachbarten Sauerstoffatomen enthaltende Alkylidendiyl-Gruppe substituiert ist, wobei ein 5- oder 6-Ringketal gebildet wird und die jeweils gegebenenfalls einfach oder zweifach durch Methyl subsitutiert sein können, oder
- A, B: und das Kohlenstoffatom, an das sie gebunden sind, stehen besonders bevorzugt für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄-Alkendiyl oder Butadiendiyl stehen,
- G: steht besonders bevorzugt für Wasserstoff (a) oder für eine der Gruppen 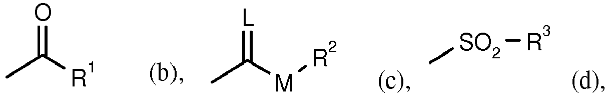 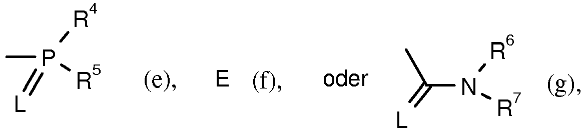
in welchen
E für ein Metallion oder ein Ammoniumion steht,
L für Sauerstoff oder Schwefel steht und
M für Sauerstoff oder Schwefel steht,
R¹ steht besonders bevorzugt für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl,
   für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
   für jeweils gegebenenfalls einfach durch Chlor, Brom oder Methyl substituiertes Furanyl, Thienyl oder Pyridyl,
R² steht besonders bevorzugt für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl,
   für Cyclopentyl oder Cyclohexyl
   oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl,
R³ steht besonders bevorzugt für jeweils gegebenenfalls einfach bis dreifach durch Fluor oder Chlor substituiertes Methyl, Ethyl, Propyl oder iso-Propyl, oder jeweils gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl,
R⁴ und R⁵ stehen unabhängig voneinander besonders bevorzugt für C₁-C₄-Alkoxy oder C₁-C₄-Alkylthio oder für jeweils gegebenenfalls einfach durch Fluor, Chlor, Brom, Nitro, Cyano, Methyl, Methoxy, Trifluormethyl oder Trifluormethoxy substituiertes Phenyl, Phenoxy oder Phenylthio,
R⁶ und R⁷ stehen unabhängig voneinander besonders bevorzugt für Wasserstoff, für C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für gegebenenfalls einfach bis zweifach durch Fluor, Chlor, Brom, Methyl, Methoxy oder Trifluormethyl substituiertes Phenyl, oder zusammen für einen C₅-C₆-Alkylenrest, in welchem gegebenenfalls eine Methylengruppe durch Sauerstoff oder Schwefel ersetzt ist.
W steht ganz besonders bevorzugt für Wasserstoff, Chlor, Methyl, Ethyl oder Methoxy, (hervorgehoben für Wasserstoff, Chlor oder Methyl),
X steht ganz besonders bevorzugt für Wasserstoff, Chlor, Methyl, Ethyl, Methoxy oder Ethoxy, (hervorgehoben für Wasserstoff, Chlor oder Methyl),
Y und Z stehen ganz besonders bevorzugt unabhängig voneinander für Wasserstoff, Chlor, Methyl oder für den Rest 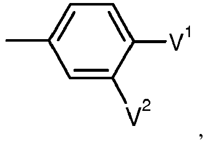
wobei in diesem Falle nur einer der Reste Y oder Z für 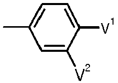 stehen darf, (hervorgehoben steht Z für 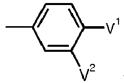 und Y für Wasserstoff)
- V¹: steht ganz besonders bevorzugt für Wassersoff, Fluor oder Chlor, (hervorgehoben für Wasserstoff oder Chlor),
- V²: steht ganz besonders bevorzugt für Wasserstoff, Fluor oder Chlor, (hervorgehoben für Wasserstoff),
- A: steht ganz besonders bevorzugt für Methyl, Ethyl, Propyl, iso-Propyl oder Cyclopropyl, (hervorgehoben für Methyl),
- B: steht ganz besonders bevorzugt für Wasserstoff oder Methyl, (hervorgehoben für Methyl),
- A, B: und das Kohlenstoffatom an das sie gebunden sind, stehen ganz besonders bevorzugt für gesättigtes C₅-C₆-Cycloalkyl, in welchem gegebenenfalls ein Ringglied durch Sauerstoff ersetzt ist und welches gegebenenfalls einfach durch Fluor, Chlor, Methyl, Ethyl, Methoxymethyl, Methoxy, Ethoxy, Propoxy, Butoxy, Trifluorethoxy substituiert ist, (hervorgehoben für C₆-Cycloalkyl, welches durch Methoxy substituiert ist), oder
- A, B: und das Kohlenstoffatom, an das sie gebunden sind, stehen ganz besonders bevorzugt für C₆-Cycloalkyl, welches gegebenenfalls durch eine gegebenenfalls durch eine mit Sauerstoff unterbrochene Alkylidendiyl-Gruppe oder durch eine mit zwei nicht direkt benachbarten Sauerstoffatomen enthaltende Alkylidendiyl-Gruppe substituiert ist, wobei ein 5- oder 6-Ringketal gebildet wird, die jeweils einfach oder zweifach durch Methyl substituiert sein können,
- G: steht ganz besonders bevorzugt für Wasserstoff (a) oder für eine der Gruppen 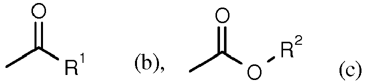
oder
E(f)
in welchen
- E: für ein Lithium-, Natrium-, Kalium-, Rubidium-, Cäsium-, Magnesium-, Calciumion oder ein Ammoniumion steht,
- R¹: steht ganz besonders bevorzugt für Methyl, Ethyl, Propyl, iso-Propyl, Butyl, iso-Butyl, sek.-Butyl, tert.-Butyl, Cyclopropyl, Cyclopentyl oder Cyclohexyl,
- R²: steht ganz besonders bevorzugt für Methyl, Ethyl, Propyl, iso-Propyl, Butyl, iso-Butyl, sek.-Butyl, tert.-Butyl, oder für Benzyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Die beim Verfahren (A*) als Ausgangsstoffe benötigten Verbindungen der Formel (II) 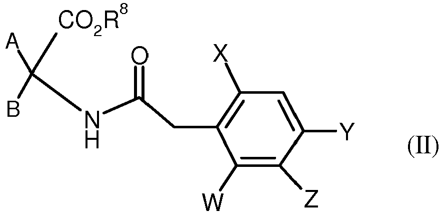 in welcher
A, B, W, X, Y, Z und R⁸ die oben angegebenen Bedeutungen haben,
erhält man beispielsweise, wenn man Aminosäurederivate der Formel (VI) 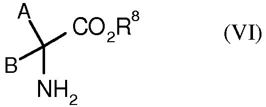 in welcher
A, B und R⁸ die oben angegebene Bedeutung haben,
mit substituierten Phenylessigsäurederivaten der Formel (VII) 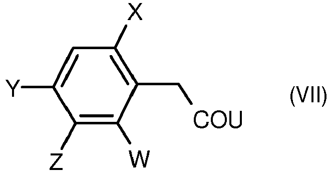 in welcher
W, X, Y und Z die oben angegebenen Bedeutungen haben und
U für eine durch Carbonsäureaktivierungsreagenzien wie Carbonyldiimidazol, Carbonyldiimide (wie z.B. Dicyclohexylcarbondiimid), Phosphorylierungsreagenzien (wie z.B. POCl₃, BOP-Cl), Halogenierungsmittel wie z.B. Thionylchlorid, Oxalylchlorid oder Phosgen sowie durch Benzolsulfonylchlorid oder Chlorameisensäureester eingeführte Abgangsgruppe steht,
acyliert (Chem. Reviews 52, 237-416 (1953); Bhattacharya, Indian J. Chem. 6, 341-5, 1968)
   oder wenn man Acylaminosäuren der Formel (VIII) 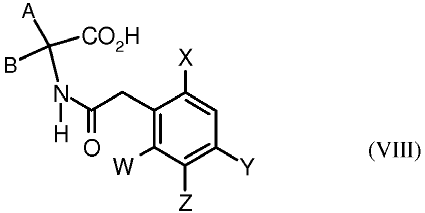 in welcher
   A, B, W, X, Y und Z die oben angegebenen Bedeutungen haben,
   verestert (Chem. Ind. (London) 1568 (1968)).

Man erhält die Verbindungen der Formel (VIII) beispielsweise, wenn man Aminocarbonsäuren der Formel (IX) 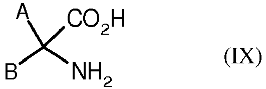 in welcher
A und B die oben angegebenen Bedeutungen haben
mit substituierten Phenylessigsäurederivaten der Formel (VII) 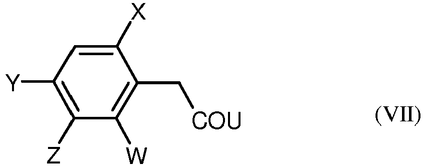 in welcher
U, W, X, Y und Z die oben angegebenen Bedeutungen haben
z.B. nach Schotten-Baumann acyliert (Organikum, VEB Deutscher Verlag der Wissenschaften, Berlin 1977, S. 505).

Die Verbindungen der Formeln (VI) und (IX) sind bekannt und lassen sich nach bekannten Verfahren darstellen (siehe z.B. Compagnon, Ann. Chim. (Paris) [14] 5, S. 11-22, 23-27 (1970), L. Munday, J. Chem. Soc. 4372 (1961); J.T. Eward, C. Jitrangeri, Can. J. Chem. 53, 3339 (1975), WO 02/02532 sowie wie in den eingangs zitierten Offenlegungsschriften beschrieben.

Für diese bekannten Verfahren werden also in jedem Fall substituierte Phenylessigsäurederivate der Formel (VII) 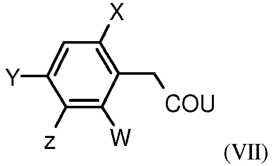 in welcher U, W, X, Y und Z die oben angegebenen Bedeutungen haben,
benötigt.

Die Verbindungen der Formel (VII) sind aus den eingangs zitierten Offenlegungsschriften wie z. Bsp. WO 98/05638, WO 01/74770 bekannt oder lassen sich nach den dort beschriebenen Verfahren herstellen. Diese Verfahren sind teilweise technisch sehr aufwendig, vielstufig oder mit geringen Totalausbeuten belastet.

Es bestand daher weiterhin Bedarf an neuen Verfahren zur Herstellung von Verbindungen der allgemeinen Formel (I) 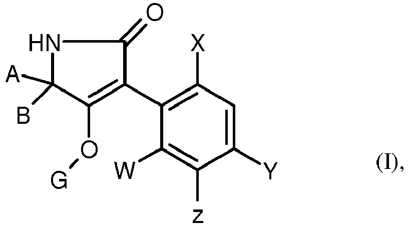 in welcher
A, B, W, X, Y, Z und G die oben angegebenen Bedeutungen haben, wobei X zusätzlich auch für Wasserstoff stehen darf, unter Vermeidung der Verwendung von Phenylessigsäurederivaten der Formel (VII).

Die u.g. neuen Verbindungen der Formel (X) sind Zwischenverbindungen zur Herstellung von Verbindungen der Formel (I) nach dem im folgenden beschriebenen Verfahren:
- (Aα): Verfahren zur Herstellung von Verbindungen der Formel (I), dadurch gekennzeichnet, dass man in einem ersten Schritt Verbindungen der Formel (X) 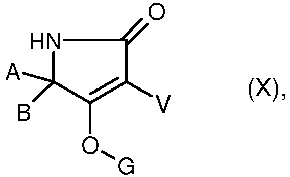
in welcher
- A und B: die oben angegebenen Bedeutungen haben,
- G: für die oben angegebenen Gruppen a), b), c) und E steht,
- V: für Wasserstoff steht oder
- (Aß) V: für COOR⁸ steht,
wobei R⁸ für Alkyl (bevorzugt für C₁-C₈-Alkyl) steht
und A, B und G die oben angegebenen Bedeutungen haben,
mit einer Verbindung der Formel (XI) 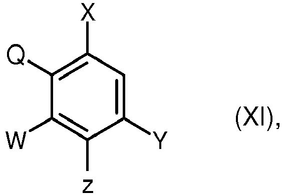 in welcher
W, X, Y und Z die oben angegebenen Bedeutungen haben mit der Ausnahme, dass als Halogen jetzt nur noch Fluor und Chlor stehen können, X auch zusätzlich für Wasserstoff stehen darf und
Q für Triflat, Brom oder Iod steht, bevorzugt für Brom oder Jod,
   in Gegenwart einer Base, eines Palladiumkatalysators und eines Phosphinliganden der
   Formel (XII') 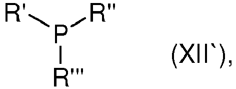 in welcher die Reste
   R', R" und R'" unabhängig voneinander für C₁ - C₁₂-Alkyl, C₅ - C₁₀- Cycloalkyl, C₆ - C₁₀-Aryl, die gegebenenfalls einfach oder mehrfach substituiert durch C₁ - C₆-Alkyl, C₁ -C₆-Alkoxy, C₁ - C₆-Alkylamino, C₁ - C₆-Dialkylamino sein können oder gegebenenfalls einfach oder mehrfach durch C₁ - C₆-Alkyl, C₁ - C₆-Alkoxy, C₁ - C₆-Alkylamino oder C₁ - C₆-Dialkylamino substituiertes Phenyl stehen,
   in einem Verdünnungsmittel umsetzt.

Als Basen kommen für das erfindunsgemäße Verfahren allgemein bekannte organische und anorganische Basen zum Einsatz. Als organische Basen seien beispielhaft Trimethylamin, Triethylamin, Tributylamin, Diisopropylamin, Diisopropylethylamin, N,N-Dimethylanilin, DABCO, DBU, Pyridin, Picoline, Luitidine, 5-Ethyl-2-methyl-pyridin genannt. Als anorganische Basen seien beispielhaft Alkali- und Erdalkalihydroxide wie LiOH, NaOH, KOH, Mg(OH)₂ und Ca(OH)₂, Alkalialkoholate wie NaOMe, NaOEt, NaOtert.butyl, KOtert.butyl, Alkali- und Erdalkalicarbonate wie Na₂CO₃, K₂CO₃, Cs₂CO₃ und CaCO₃ Alkali- und Erdalkalihydrogencarbonate wie NaHCO₃, KHCO₃, Alkali- und Erdalkaliphosphate wie Na₃PO₄, K₃PO₄ und Mg₃(PO₄)₂, Alkali- und Erdalkalihydrogenphosphate wie Na₂HPO₄, K₂HPO₄ und BaHPO₄, Alkali- und Erdalkalidihydrogenphosphate wie NaH₂PO₄, KH₂PO₄ und Ca(H₂PO₄)₂, Alkali- und Erdalkalihydride wie NaH, KH und CaH₂ und Alkali- und Erdalkaliamide wie NaNH₂, KNH₂ und LiNPr₂ genannt.

Bevorzugt sind die Alkali- und Erdalkalicarbonate und -phosphate.

Im erfindunsgemäße Verfahren kann die eingesetzte Basenmenge in weiten Bereichen variiert werden. Für gewöhnlich wird man jedoch mindestens ein Moläquivalent Base bezogen auf die Verbindung der allgemeinen Formel (X) einsetzen. Es ist auch möglich, die Base in Überschüssen von 1,1 bis 15, bevorzugt 1,1 bis 6 Moläquivalenten Base bezogen auf die Verbindung der allgemeinen Formel (X) einzusetzen.

Als Palladiumkatalysatoren für das erfindunsgemäße Verfahren kommen grundsätzlich alle Palladiumverbindungen in Frage, aus denen unter den Reaktionsbedingungen *in situ* ein aktiver Katalysator enstehen kann. Beispielhaft seien hier genannt: Pdalladiumchlorid, Palladiumbromid, Palladiumiodid, Palladiumacetat, Palladiumtrifluoracetat, Palladiumnitrat, Palladiumsulfat, Palladiumacetylacetonat, Allylpalladiumchlorid-Dimer, Bis(dibenzylidenaceton)-palladium, Bis(triphenylphosphin)-palladium(II)chlorid, Bis(triphenylphosphin)palladium(II)bromid, Tetrakis(triphenylphosphin)palladium(0), Bis(acetonitril)palladiumdichlorid, Bis(benzonitril)-palladiumdichlorid, 1,1'-Bis(diphenylphosphino)ferrocen-palladiumdichlorid, Di-µ-chlorobis(tri-tert.butylphosphino)-dipalladium(I), Di-µ-bromobis(tri-tert.butylphosphino)-dipalladium(I), metallisches Palladium wie Palladiumschwarz oder Palladiumpulver, oder Palladium auf verschiedenen Trägern wie beispielsweise Palladium auf Aktivohle, Palladium auf Bariumsulfat, Palladium auf Calciumcarbonat oder Palladium auf Aluminiumoxid.

Die im erfindungsgemäßen Verfahren einzusetzende Menge an Palladiumkatalysator kann innerhalb weiter Grenzen variiert werden. Für gewöhnlich wird man die geringstmögliche Menge, mit der noch eine gute Ausbeute erzielt wird, einsetzen. Typischerweise liegt die Menge an Palladiumkatalysator zwischen 0,001 und 10 Molprozent, bezogen auf die Verbindung der allgemeinen Formel (X). Bevorzugt werden Mengen von 0,01 bis 5 Molprozent eingesetzt.

Als Verdünnungsmittel für das erfindunsgemäße Verfahren können im Prinzip alle unter den Reaktionsbedingungen inerten organischen Lösungsmittel verwendet werden. Beispielhaft seien genannt: Ether wie Diethylether, Methyl-tert.butylether, Methyl-cyclopentylether, Tetrahydrofuran, 2-Methyltetrahydrofuran, 1,4-Dioxan; Kohlenwasserstoffe wie Toluol, Xylole, Mesitylen, Chlorbenzol, 1,2-Dichlorbenzol; Amide wie N,N-Dimethylformamid, N,N-Dimethylacetamid, N-Methyl-pyrrolidon; Dimethylsulfoxid oder Sulfolan.

Im erfindungsgemäßen Verfahren können unterschiedlichste Phosphinliganden der allgemeinen Formel (XII') eingesetzt werden. Beipielhaft seien genannt: Triphenylphosphin, Tri-ortho-tolyl-phosphin, Tri-meta-tolyl-phosphin, Tri-para-tolyl-phosphin,

Benzyl-di-1-adamantylphosphin (cataCXium ABn), Dinatrium-bis(4,6-dimethyl-3-sulfonatophenyl)-(2,4dimethylphenyl)-phosphin, Trinatrium-tris(4,6-dimethyl-3-sulfonato-phenyl)phosphin, Butyl-di-1-adamantylphosphin (cataCXium A), Tributylphosphin, Tricyclohexylphosphin, Tri-tert.butylphosphin, 2-Di-tert.butylphosphino-1,1'-binaphthyl, 2-Di-tert.butylphosphino-1,1'-biphenyl, 2-Di-cyclohexylphosphino-biphenyl, 2-Di-tert.butylphosphino-2'-(N,N-dimethylamino)biphenyl, 2-Di-cyclohexylphosphino-2'-(N,N-dimethylamino)biphenyl, 2-Di-tert.butylphosphino-2' -methylbiphenyl, 2-Di-cyclohexylphosphino-2' -methylbiphenyl, 2-Di-cyclohexylphosphino-2' -isopropylbiphenyl, 2-Di-tert.butylphosphino-2',4',6'-tri-isopropyl-1,1'-biphenyl, 2-Di-cyclohexylphosphino-2' ,4' ,6' -tri-isopropyl-1,1' -biphenyl, 2-Diphenylphosphino-2'-(N,N-dimethylamino)biphenyl, 2-Dicyclohexylphosphino-2',6'-di-iso-propoxy-1,1'-biphenyl (RuPhos), N-(2-Methoxyphenyl)-2-(di-tert.butylphosphino)pyrrol, N-Phenyl- 2-(di-tert.butyl-phosphino)-pyrrol, 9,9-Dimethyl-4,5-bis(diphenylphosphino)xanthen (XANTPHOS), 9,9-Dimethyl-4,5-bis(di-tert.butylphosphino)xanthen, Bis(2-diphenylphosphinophenyl)ether (DPEphos), 2,2'-Bis(diphenylphosphino)-1,1'-biphenyl (BIPHEP), 2,2'-Bis(diphenylphosphino)-1,1'-binaphthyl (BINAP), 1,1'-Bis(diphenylphosphino)-ferrocen (DPPF).

Die im erfindungsgemäßen Verfahren einzusetzende Menge an Phosphinliganden der allgemeinen Formel (XII') liegt zwischen 0,25 und 5 Mol pro Mol Palladiumkatalysator. Bevorzugt setzt man zwischen 0,5 und 2,5 Mol pro Mol ein.

Die Reaktionstemperatur für das erfindungsgemäße Verfahren kann in weiten Grenzen variiert werden. Üblicherweise arbeitet man bei einer Temperatur zwischen 20 und 200°C, bevorzugt zwischen 50 und 180°C.

Das erfindungsgemäße Verfahren wird üblicherweise bei Normaldruck unter Ausschluß von Luftsauerstoff und Feuchtigkeit ausgeführt. Das Verfahren kann aber grunsätzlich auch unter vermindertem oder erhöhtem Druck durchgeführt werden.

Bei der Durchführung des erfindungsgemäßen Verfahrens ist es möglich, die Verbindungen der Formel (XI) in einem größeren Überschuss (bis zu 10 Mol, bevorzugt bis zu 2 Mol) einzusetzen.

Einige Verbindungen der Formel (X) mit G = Wasserstoff und substituertem Benzoyl sind teilweise bekannt aus WO 94/01401 sowie der dort angegebenen Literatur und teilweise neu. Weitere Verbindungen der Formel (X) sind aus Journal of Organic Chemistry, Bd. 28, Nr. 11, 1963, Seiten 3150-3165 (Verbindung XXXVII), DE 19515690 (4-Hydroxy-5,5-(2-methyl)-pentamethylenpyrrolin-2-on (Edukt aus Beispiel 3)) und die entsprechenden Edukte der Beispiele 4 und 5) und DE 4409044 (Verbindungen aus der Tabelle 3 auf den Seiten 60-61) bekannt.

Die Verbindungen der Formel (X) 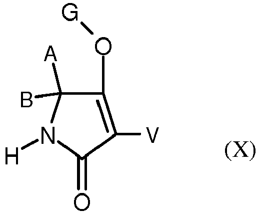 mit
- A, B: und das Kohlenstoffatom an das sie gebunden sind, stehen hervorgehoben für gesättigtes C₆-Cycloalkyl, worin ein Ringglied durch Sauerstoff ersetzt ist oder
- A, B: und das Kohlenstoffatom an das sie gebunden sind, stehen hervorgehoben für gesättigtes C₆-Cycloalkyl, welches zweifach durch Fluor oder einfach durch Methoxy substituiert ist, oder
- A, B: und das Kohlenstoffatom, an das sie gebunden sind, stehen hervorgehoben für C₆-Cycloalkyl, welches durch eine mit zwei nicht direkt benachbarte Sauerstoffatome enthaltende Alkylendiyl-Gruppe substituiert ist, die mit dem Kohlenstoffatom, an das sie gebunden ist, einen weiteren fünfgliedrigen Ring bildet,
- G: steht hervorgehoben für Wasserstoff (a) oder 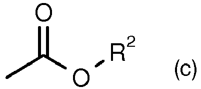
wobei R² hervorgehoben für Ethyl steht,
- V: steht hervorgehoben für Wasserstoff (X-1) oder COOR⁸ (X-2),
wobei R⁸ hervorgehoben für Methyl steht
sind neu und Kern der Erfindung.

Unter Einbeziehung der verschiedenen Bedeutungen (a) und (c) der Gruppe G ergeben sich folgende hauptsächliche Strukturen (X-1-a) und (X-1-c), wenn V für Wasserstoff steht,
**(X-1-a):** 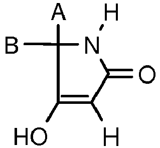 **(X-1-c):** 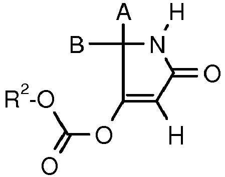 worin
A, Bund R² die oben angegebenen Bedeutungen besitzen.

Unter Einbeziehung der verschiedenen Bedeutungen (a) und (c) der Gruppe G ergeben sich folgende hauptsächliche Strukturen (X-2-a) und (X-2-c), wenn V für COOR⁸ steht,
**(X-2-a):** 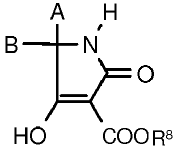 **(X-2-c):** 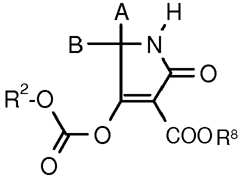 worin
A, B, R² und R⁸ die oben angegebenen Bedeutungen besitzen.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Hervorgehoben sind Verbindungen der Formel (X) in welchen G für Wasserstoff steht.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nicht anderes angegeben ist, einfach oder mehrfach substituiert sein, wobei bei Mehrfachsubstitutionen die Substituenten gleich oder verschieden sein können.

Im Einzelnen seien außer bei den Beispielen genannten Verbindungen die folgenden Verbindungen der Formle (X) mit G = H genannt:

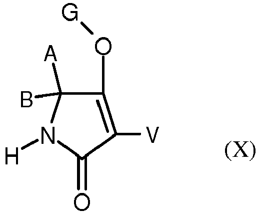

**Tabelle 1**

| **A** | **B** | **V** |
|---|---|---|
| -(CH₉)₉-O-(CH₂)₂- | | H |
| -CH₉-O-(CH₉)₃- | | H |
| -(CH₂)₂-S-(CH₂)₂- | | H |
| -CH₂-CHCH₃-(CH₂)₃- | | H |
| -CH₂-CHOCH₃-(CH₂)₂- | | H |
| -CH₂-CHOC₂H₅-(CH₂)₂- | | H |
| -CH₂-CHOC₃H₇-(CH₂)₂- | | H |
| -CH₂-CHOC₄H₉-(CH₂)₂- | | H |
| -CH₂-CHO(CH₂)₂OCH₃(CH₂)₂- | | H |
| 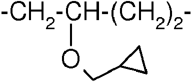 | | H |
| -CH₂-CHOCH₃-(CH₂)₃- | | H |
| -CH₂-CHOC₂H₅-(CH₂)₃- | | H |
| -CH₂-CHOC₃H₇-(CH₂)₃- | | H |
| -CH₂-CHOC₄H₉-(CH₂)₃- | | H |
| -CH₂-CHO(CH₂)₂OCH₃-(CH₂)₃- | | H |
| 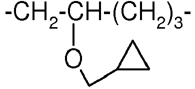 | | H |
| -(CH₂)₂-CHOCH₃-(CH₂)₂- | | H |
| -(CH₂)₂-CHOC₂H₅-(CH₂)₂- | | H |
| -(CH₂)₂-CHOC₃H₇-(CH₂)₂- | | H |
| -(CH₂)₂-CHO-CH₂CF₃-(CH₂)₂- | | H |
| -(CH₂)₂-C(CH₃)₂-(CH₂)₂- | | H |
| -CH₂-(CHCH₃)₂-(CH₂)₂- | | H |
| -(CH₂)₂-CF₂-(CH₂)₂- | | H |
| 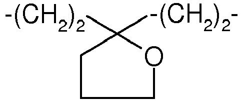 | | H |
| 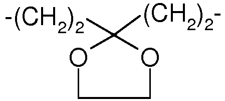 | | H |
| 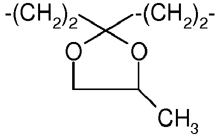 | | H |
| 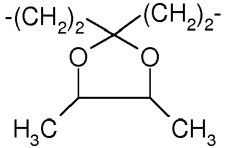 | | H |
| 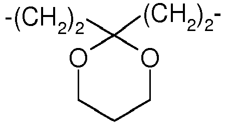 | | H |
| 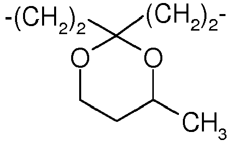 | | H |
| 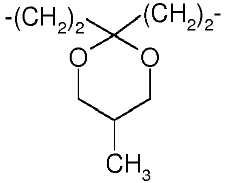 | | H |
| 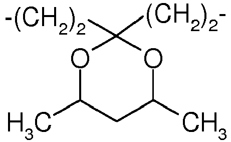 | | H |
| 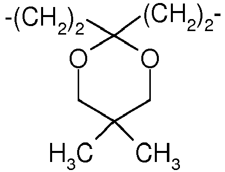 | | H |
| 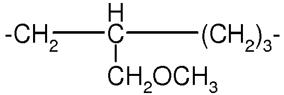 | | H |
| 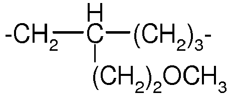 | | H |
| 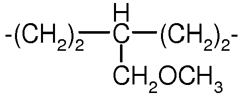 | | H |
| 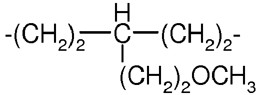 | | H |
| 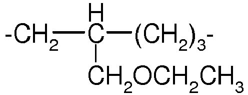 | | H |
| 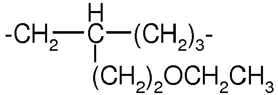 | | H |
| 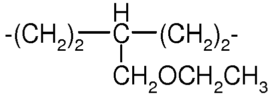 | | H |
| 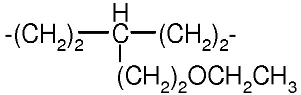 | | H |

**Tabelle 2** A und B wie in Tabelle 1 angegeben und V = COOCH₃

**Tabelle 3** A und B wie in Tabelle 1 angegeben und V = COOC₂H₅
(B) Man erhält Verbindungen der Formel (X-2-a) 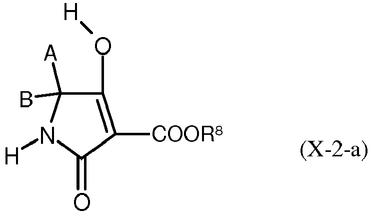 in welcher A, B und R8 die oben angegebenen Bedeutungen haben, wenn man
   Verbindungen der Formel (XII) 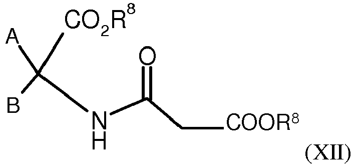 in welcher
   A, B und R⁸ die oben angegebenen Bedeutungen haben,
   in Gegenwart eines Verdünnungsmittels und in Gegenwart einer Base intramolekular kondensiert.
(C) Außerdem erhält man Verbindungen der Formel (X-1-a) 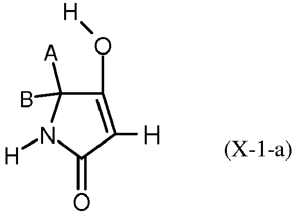 in welcher
   A und B die oben angegebenen Bedeutungen haben,
   wenn man
   Verbindungen der Formel (X-2-a) 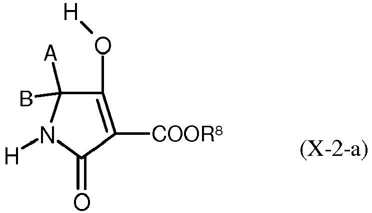 in welcher A, B und R⁸ die oben angegebenen Bedeutungen haben, hydrolysiert und anschließend decarboxyliert.

Weiterhin wurde gefunden
- ((E): dass man die Verbindungen der oben gezeigten Formeln (X-1-c) oder (X-2-c), in welchen R², A, B und V die oben angegebenen Bedeutungen haben, erhält, wenn man Verbindungen der oben gezeigten Formeln (X-1-a) und (X-2-a), in welchen A, B und V die oben angegebenen Bedeutungen haben,
mit Chlorameisensäureestern der Formel (V)

R²-O-CO-Cl (V)

in welcher
R² die oben angegebene Bedeutung hat,
gegebenenfalls in Gegenwart eines Verdünnungsmittels und gegebenenfalls in Gegenwart eines Säurebindemittels umsetzt;

Verwendet man beispielsweise gemäß Verfahren (Aα) 8-Methoxy-1-azaspiro[4,5]decan-2,4-dion und 2,5-Dimethyl-brombenzol als Ausgangsstoffe, so kann der Reaktionsverlauf durch folgendes Schema wiedergegeben werden:

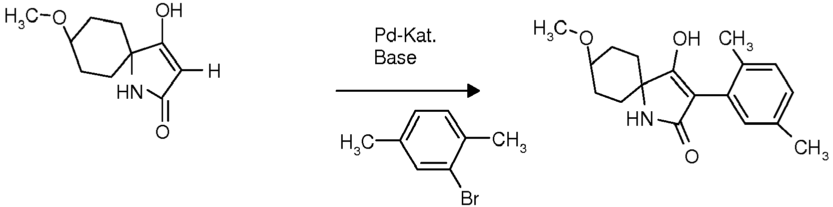

Verwendet man beispielsweise gemäß Verfahren (Aß) 3-Methoxycarbonyl-8-methoxy-1-azaspiro-[4,5]-decan-2,4-dion und 2,5-Dimethyl-brombenzol als Ausgangsverbindungen, so kann der Verlauf des erfindungsgemäßen Verfahrens durch folgendes Reaktionsschema wiedergegeben werden: 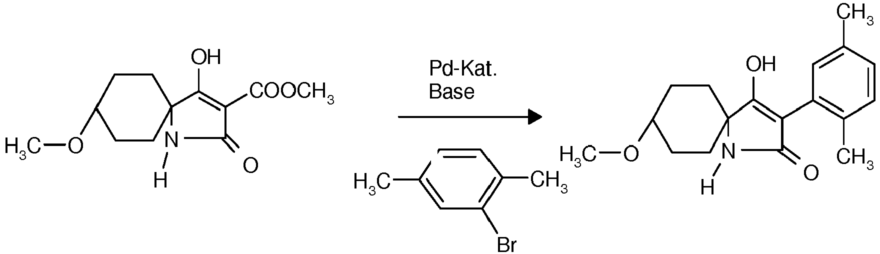 Verwendet man beispielsweise gemäß Verfahren (B) N-Ethoxycarbonylacetyl-1-amino-4-methoxy-cyclohexancarbonsäureethylester als Ausgangsstoff, so kann der Verlauf des erfindungsgemäßen Verfahrens durch folgendes Reaktionsschema wiedergegeben werden:

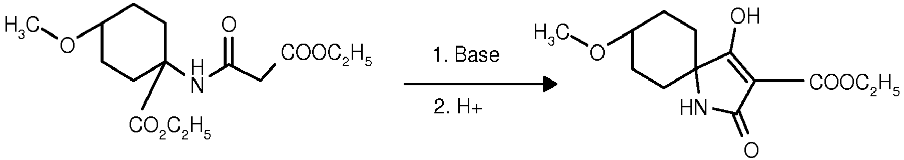

Verwendet man beispielsweise gemäß Verfahren (C) 3-Methoxycarbonyl-8-methoxy-1-azaspiro[4,5]decan-2,4-dion und einen Überschuss an wässriger Base als Ausgangsprodukte, so kann der Reaktionsverlauf durch folgendes Reaktionsschema wiedergegeben werden:

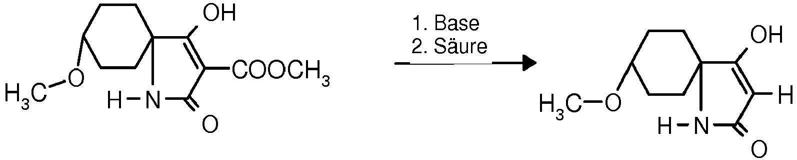

Verwendet man beispielsweise gemäß Verfahren (E) 8-Methoxy-1-azaspiro[4,5]decan-2,4-dion und Chlorameisensäureethylester als Ausgangsverbindungen, so kann der Verlauf des erfindungsgemäßen Verfahrens durch folgendes Reaktionsschema wiedergegeben werden:

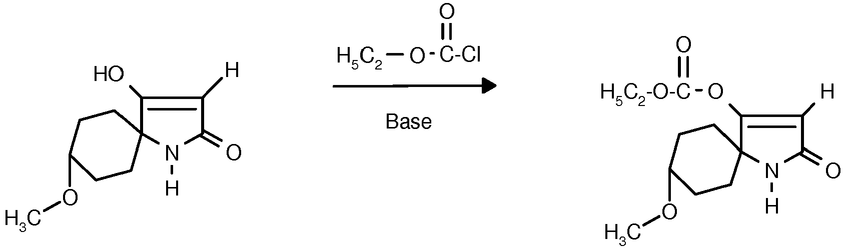

Die beim erfindungsgemäßen Verfahren (B) als Ausgangsstoffe benötigten Verbindungen der Formel (XII) 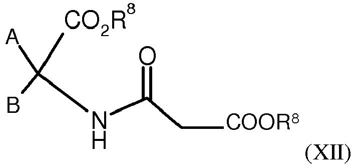 in welcher
A, B und R⁸ die oben angegebenen Bedeutungen haben,
sind neu.

Man erhält die Acylaminosäureester der Formel (XII) beispielsweise, wenn man Aminosäurederivate der Formel (XV) 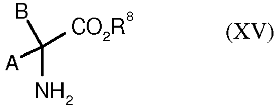 in welcher
A, B und R⁸ die oben angegebene Bedeutung haben,
mit substituierten Malonsäurehalbesterchloriden der Formel (XVI) 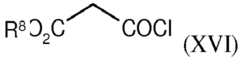 in welcher R⁸ die oben angegebenen Bedeutungen hat,
acyliert (Chem. Reviews 52, 237-416 (19953); Bhattacharya, Indian J. Chem. 6, 341-5, 1968).

Weiterhin lassen sich die bei dem obigen Verfahren (B) verwendeten Ausgangsstoffe der Formel (XII) 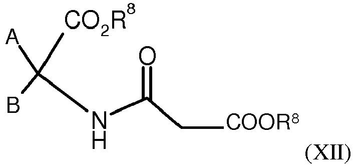 in welcher
A, B und R⁸ die oben angegebenen Bedeutungen haben,
herstellen, wenn man 1-Amino-carbonsäurenitrile der Formel (XVII) 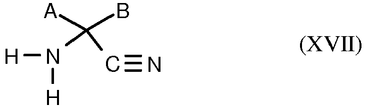 in welcher
A und B die oben angegebenen Bedeutungen haben,
mit Malonsäurehalbesterchloriden der Formel (XVI) 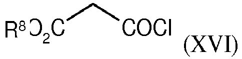 in welcher R⁸ die oben angegebene Bedeutungen hat,
zu Verbindungen der Formel (XVIII) 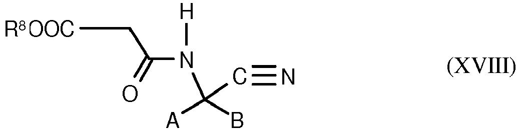 in welcher
A, B und R⁸ die oben angegebenen Bedeutungen haben,
umsetzt,
und diese anschließend einer sauren Alkoholyse unterwirft.

Die Verbindungen der Formel (XVIII) sind ebenfalls neu und lassen sich analog nach bekannten Verfahren die in der eingangs zitierten Literatur oder z.B. wie in EP-A-595 130 beschrieben sind herstellen. Die Verbindungen der Formel (XVII) sind teilweise käuflich, teilweise bekannt z.B. WO 2008/128058 und teilweise auch neu und lassen sich z.B. wie in EP-A-595 130 beschrieben herstellen.

Außerdem lassen sich die bei dem obigen Verfahren (B) verwendeten Ausgangsstoffe der Formel (XII) 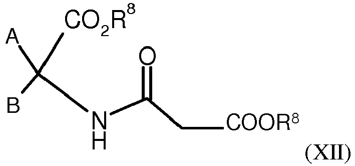 in welcher
A, B, und R⁸ die oben angegebenen Bedeutungen haben,
herstellen, wenn man 1-Aminocarbonsäurenitrile der Formel (XVII) 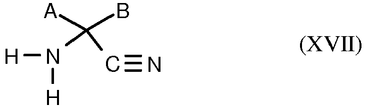 in welcher
A und B die oben angegebenen Bedeutungen haben,
mit Cyanessigsäure der Formel (XIX) 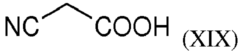 zu Verbindungen der Formel (XX) 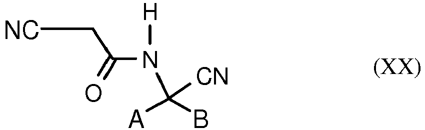 in welcher
A und B die oben angegebenen Bedeutungen haben,
umsetzt,
und diese anschließend einer sauren Alkoholyse unterwirft.

Die Verbindungen der Formel (XX) sind ebenfalls neu und lassen sich analog nach bekannten Verfahren die in der eingangs zitierten Literatur beschrieben sind herstellen.

Die zur Durchführung des erfindungsgemäßen Verfahren (E) außerdem als Ausgangsstoffe benötigten Chlorameisensäureester der Formel (V) sind allgemein bekannte Verbindungen der organischen bzw. anorganischen Chemie.

Die Verbindungen der Formeln (XV) und (XVII) sind darüber hinaus aus den eingangs zitierten Patentanmeldungen bekannt und/oder lassen sich nach den dort angegebenen Methoden herstellen.

Die Verbindungen der Formeln (XVI) und (XIX) sind käuflich.

Das Verfahren (B) ist dadurch gekennzeichnet, dass man Verbindungen der Formel (XII), in welcher A, B und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (=Methyltrialkyl(C₈-C₁₀)ammoniumchlorid) oder TDA 1 (=Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (B) innerhalb eines größeren Bereiches variiert werden. Im allgemeinen arbeitet man bei Temperaturen zwischen -75°C und 200°C, vorzugsweise zwischen -50°C und 150°C. Das erfindungsgemäße Verfahren (A) wird im allgemeinen unter Normaldruck durchgeführt.

Bei der Durchführung des erfindungsgemäßen Verfahrens (B) setzt man die Reaktionskomponente der Formel (XII) und die deprotonierende Base im allgemeinen in äquimolaren bis etwa doppeltäquimolaren Mengen ein. Es ist jedoch auch möglich, die eine oder andere Komponente in einem größeren Überschuss (bis zu 3 Mol) zu verwenden.

Das Verfahren (C) ist dadurch gekennzeichnet, dass man Verbindungen der Formel (X-2), in welcher A, B und R⁸ die oben angegebenen Bedeutungen haben, in Gegenwart eines Verdünnungsmittels und gegebenenfalls in Gegenwart einer Base oder Säure hydrolysiert und decarboxyliert.

Als Verdünnungsmittel können bei dem erfindungsgemäßen Verfahren (C) alle gegenüber den Reaktionsteilnehmern inerten organischen Solventien eingesetzt werden. Vorzugsweise verwendbar sind Ether, wie Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol, aber auch Wasser.

Als Base können bei der Durchführung des erfindungsgemäßen Verfahrens (C) alle üblichen laugenbildenden Basen eingesetzt werden. Vorzugsweise verwendbar sind Alkalimetall- und Erdalkalimetalloxide, -hydroxide und -carbonate, wie Lithiumhydroxid, Natriumhydroxid, Kaliumhydroxid, Magnesiumoxid, Calciumoxid, Natriumcarbonat, Kaliumcarbonat und Calciumcarbonat einsetzbar.

Als Säuren können bei der Durchführung des erfindungsgemäßen Verfahrens (C) alle üblichen anorganischen und organischen Säuren eingesetzt werden. Vorzugsweise verwendbar sind als anorganische Säuren z.B. Salzsäure, Schwefelsäure, Phosphorsäure und Salpetersäure. Vorzugsweise verwendbar sind als organische Säuren z.B. Ameisensäure, Essigsäure, Trifluoressigsäure, Oxalsäure, Zitronensäure und deren wässrige Lösungen.

Als Besonderheit können die bei dem Verfahren (C) eingesetzten Verbindungen der Formel (X-2) auch autokatalytisch als Säure eingesezt werden.

Die Reaktionstemperatur kann bei der Durchführung des erfindungsgemäßen Verfahrens (C innerhalb eines größeren Bereiches variiert werden. Im allgemeinen arbeitet man bei Temperaturen zwischen -20°C und 200°C, vorzugsweise zwischen 0°C und 150°C. Das erfindungsgemäße Verfahren (C) wird im allgemeinen unter Normaldruck durchgeführt.

Bei der Durchführung des erfindungsgemäßen Verfahrens (C) setzt man die Reaktionskomponente der Formel (X-2) und die Base oder die Säure im allgemeinen in äquimolaren bis etwa doppeltäquimolaren Mengen ein. Es ist jedoch auch möglich, die Base oder die Säure in einem größeren Überschuss aber auch katalytisch zu verwenden.

Das Verfahren (E) ist dadurch gekennzeichnet, dass man Verbindungen der Formeln (X-1) oder (X-2) jeweils mit Chlorameisensäureestern der Formel (V) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (E) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (E) alle gegenüber den Chlorameisensäureestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (E) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (E) wird im allgemeinen unter Normaldruck durchgeführt.

Bei der Durchführung des erfindungsgemäßen Verfahrens (E) werden die Ausgangsstoffe der Formeln (X-1) oder (X-2) und der entsprechende Chlorameisensäureester der Formel (V) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im allgemeinen geht man so vor, dass man ausgefallene Salze entfernt und das verbleibende Reaktionsgemisch durch Abziehen des Verdünnungsmittels einengt.

### Herstellungsbeispiele

Anmerkung: Me steht für Methyl; Et steht für Ethyl

### Beispiel 1: 5,5-Dimethyl-3-phenylpyrrolidin-2,4-dion

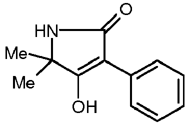

Unter Argon werden in einer ausgeheizten Apparatur 23 mg Pd(OAc)₂, 69 mg Di-tert-butyl(2'-methylbiphenyl-2-yl)phosphin und 2,44 g K₃PO₄ in 15 ml luftfreiem Dioxan vorgelegt. Man gibt 763 mg 5,5-Dimethylpyrrolidin-2,4-dion und 785 mg Brombenzol hinzu und rührt 16 Stunden unter Rückfluß. Danach lässt man auf Raumtemperatur abkühlen, verdünnt mit 20 ml Methanol, filtriert und wäscht den Filterrückstand mit 10 ml MeOH nach. Die vereinigten Filtrate werden am Rotationsverdampfer eingeengt. Der Rückstand wird in 20 ml Wasser aufgenommen und mit verdünnter Salzsäure schwach sauer gestellt. Der ausgefallene Feststoff wird abgesaugt und mit 10 ml Wasser gewaschen. Dann wird er mit Aceton vom Filter gewaschen und das Filtrat eingeengt. Man erhält 1,01 g Feststoff mit einer Reinheit von 93,8% nach GC/MS.
GC/MS: m/e = 203 (M⁺, 20%), 118 (M - NHCMe₂CO, 100%).
¹H-NMR (400 MHz, d-DMSO): δ = 1,35 (s, 6H), 7,13-7,17 (m, 1H), 7,28-7,32 (m, 2H), 7,65 (s, 1H), 7,91-7,93 (m, 2H), 11,08 (s, 1H) ppm.

### Beispiel 2: 5,5-Dimethyl-3-(2-methylphenyl)pyrrolidin-2,4-dion

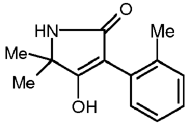

Man verfährt wie in Beispiel 1, mit dem Unterschied, dass 855 mg 2-Bromtoluol anstelle von Brombenzol eingesetzt werden. Man erhält 0,84 g Feststoff mit einer Reinheit laut GC/MS von 86,3%.
GC/MS: m/e = 217 (M⁺, 30%), 132 (M - NHCMe₂CO, 100%).

### Beispiel 3: 5,5-Dimethyl-3-(3-methylphenyl)pyrrolidin-2,4-dion

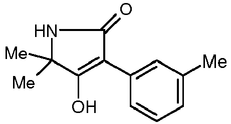

Man verfährt wie in Beispiel 1, mit dem Unterschied, dass 855 mg 3-Bromtoluol anstelle von Brombenzol eingesetzt werden. Man erhält 1,17 g Feststoff mit einer Reinheit laut GC/MS von 92%.
GC/MS: m/e = 217 (M⁺, 20%), 132 (M - NHCMe₂CO, 100%).

### Beispiel 4: 5,5-Dimethyl-3-(3-methylphenyl)pyrrolidin-2,4-dion

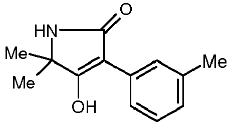

Unter Argon werden in einer ausgeheizten Apparatur 1,0 g festes Natriumhydroxid (in Form sogn. "Micropills") und 15 ml wasser- und luftfreies N-Methyl-pyrrolidon (NMP) vorgelegt. Unter Rühren gibt man dann 1,907 g 5,5-Dimethylpyrrolidin-2,4-dion hinzu und rührt 20 Minuten bei Raumtemperatur. Anschließend setzt man 1,71 g 3-Bromtoluol zu und erhitzt das Reaktionsgemisch auf 125°C. Bei dieser Temperatur gibt man dann 0,328 g Triphenylphosphin und 89 mg PdCl₂ hinzu. Man rührt 4 Stunden bei 125°C, lässt auf Raumtemperatur abkühlen, rührt das Reaktionsgemisch in 20 ml Eiswasser ein und stellt mit verdünnter Salzsäure auf pH 2. Man gibt 20 ml Methylenchlorid hinzu, verrührt, trennt die Phasen und schüttelt die wässrige Phase noch zweimal mit je 10 ml Methylenchlorid aus. Die vereinigten organischen Phasen werden getrocknet und dann am Rotationsverdampfer eingeengt. Es resultieren 1,82 g Zielprodukt (entsprechend einer Ausbeute von 84% der Theorie).

### Beispiel 5: 3-(4-Chlor-2-methylphenyl)-5,5-dimethylpyrrolidin-2,4-dion

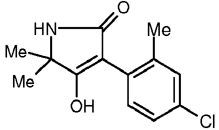

Man verfährt wie in Beispiel 1, mit dem Unterschied, dass 1,03 g 2-Brom-5-chlor-toluol anstelle von Brombenzol eingesetzt werden. Man erhält 1,37 g Feststoff mit einer Reinheit laut GC/MS von 94,3%.
GC/MS: m/e = 251 (M⁺ für ³⁵Cl, 25%), 166 (M - NHCMe₂CO, 100%).

### Beispiel 6: 3-(Biphenyl-3-yl)-5,5-dimethylpyrrolidin-2,4-dion

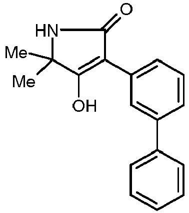

Man verfährt wie in Beispiel 1, mit dem Unterschied, dass 1,166 g 3-Brom-biphenyl anstelle von Brombenzol eingesetzt werden. Man erhält 1,52 g Feststoff mit einer Reinheit laut GC/MS von 95,4%.
GC/MS: m/e = 279 (M⁺, 35%), 194 (M - NHCMe₂CO, 90%), 165 (100%).

### Beispiel 7: 3-(2,5-Dimethylphenyl)-5,5-dimethylpyrrolidin-2,4-dion

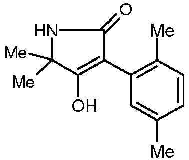

Man verfährt wie in Beispiel 1, mit dem Unterschied, dass 0,926 g 2,5-Dimethyl-brombenzol anstelle von Brombenzol eingesetzt werden. Man erhält 1,21 g Feststoff mit einer Reinheit laut GC/MS von 90%.
GC/MS: m/e = 231 (M⁺, 20%), 146 (M - NHCMe₂CO, 100%).

### Beispiel 8: 8-Methoxy-3-phenyl-1-azaspiro[4.5]decan-2,4-dion

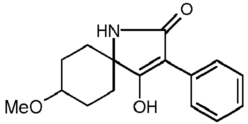

Man verfährt wie in Beispiel 1, mit dem Unterschied, dass 1,18 g 8-Methoxy-1-azaspiro[4.5]decan-2,4-dion anstelle von 5,5-Dimethylpyrrolidin-2,4-dion eingesetzt werden. Man erhält etwa 336 mg der Titelverbindung.
GC/MS: m/e = 273 (M⁺, 15%), 241 (M - MeOH, 5%), 118 (PhCHCO; 100%).

### Beispiel 9: 2,2-Dimethyl-5-oxo-4-phenyl-2,5-dihydro-1H-pyrrol-3-yl-ethylcarbonat

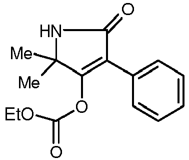

Man verfährt wie in Beispiel 1, mit dem Unterschied, dass 1,195 g 2,2-Dimethyl-5-oxo-2,5-dihydro-1H-pyrrol-3-yl-ethylcarbonat anstelle von 5,5-Dimethylpyrrolidin-2,4-dion eingesetzt werden. Man erhält die Titelverbindung in einer Ausbeute von 69% der Theorie.
GC/MS: m/e = 275 (M⁺, 2%), 203 (M-72, 80%), 188 (100%), 145 (95%), 118 (M - EtOCO, - NHCMe₂CO, 70%), 89 (100%).

### Beispiel 10: 2,2-Dimethyl-5-oxo-4-phenyl-2,5-dihydro-1H-pyrrol-3-ylacetat

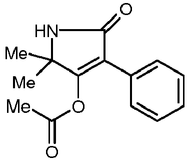

Man verfährt wie in Beispiel 1, mit dem Unterschied, dass 1,015 g 2,2-Dimethyl-5-oxo-2,5-dihydro-1H-pyrrol-3-ylacetat anstelle von 5,5-Dimethylpyrrolidin-2,4-dion eingesetzt werden. Man erhält die Titelverbindung in einer Ausbeute von etwa 35% der Theorie. Zusätzlich erhält man durch *in situ* Abspaltung des Acetylrestes 5,5-Dimethyl-3-phenylpyrrolidin-2,4-dion in einer Ausbeute von etwa 38% der Theorie.
GC/MS: m/e = 245 (M⁺, 2%), 203 (M-42, 100%), 188 (60%), 118 (80%), 43 (50%).

### Beispiel 11: 8-Methoxy-3-phenyl-1-azaspiro[4.5]decan-2,4-dion

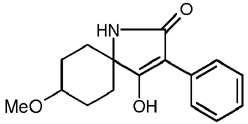

Man verfährt wie in Beispiel 1, mit dem Unterschied, dass 1,532 g Methyl-8-methoxy-2,4-dioxo-1-azaspiro[4.5]decan-3-carboxylat gemäß Beispiel (X-2-a-1) anstelle von 5,5-Dimethylpyrrolidin-2,4-dion eingesetzt werden. Man erhält die Titelverbindung in einer Ausbeute von etwa 90% der Theorie.

### Beispiel 12: 3-(4-Chlor-2-methylphenyl)-8-methoxy-1-azaspiro[4.5]decan-2,4-dion

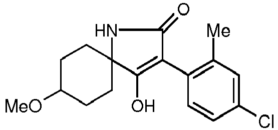

Man verfährt wie in Beispiel 11, mit dem Unterschied, dass 1,03 g 2-Brom-5-chlortoluol anstelle von Brombenzol eingesetzt werden. Man erhält die Titelverbindung in einer Ausbeute von etwa 22% der Theorie.
GC/MS: m/e = 321 (M⁺ für ³⁵Cl, 20%), 290 (M-31, 20%), 166 (100%).

### Beispiel 13: 3-(2,5-Dimethylphenyl)-8-methoxy-1-azaspiro[4.5]decan-2,4-dion

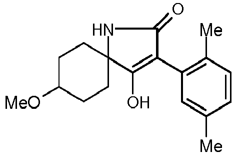

Man verfährt wie in Beispiel 11, mit dem Unterschied, dass 0,925 g 2,5-Dimethyl-brombenzol anstelle von Brombenzol eingesetzt werden. Man erhält die Titelverbindung in einer Ausbeute von etwa 20% der Theorie.
GC/MS: m/e = 301 (M⁺ 20%), 270 (M-31, 20%), 146 (100%).

### Beispiel 14: 3-[3-(4-Chlorphenyl)-6-methyl-phenyl]-5,5-dimethylpyrrolidin-2,4-dion

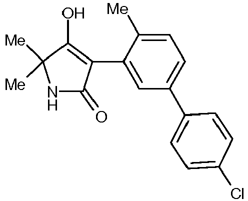

Unter Argon werden in einer ausgeheizten Apparatur 7,4 mg Pd(OAc)₂, 22 mg Di-tert-butyl(2'-methylbiphenyl-2-yl)phosphin und 0,78 g K₃PO₄ in 4,8 ml luftfreiem Dioxan vorgelet. Man gibt 203 mg 5,5-Dimethylpyrrolidin-2,4-dion und 659 mg 3-(4-Chlorphenyl)-6-methyl-brombenzol hinzu und rührt 16 Stunden unter Rückfluß. Danach lässt man auf Raumtemperatur abkühlen, verdünnt mit ca. 6 ml Methanol, filtriert und wäscht den Filterrückstand mit ca. 3 ml MeOH nach. Die vereinigten Filtrate werden am Rotationsverdampfer eingeengt. Der Rückstand wird in ca. 6 ml Wasser aufgenommen und mit 1 N Salzsäure schwach sauer gestellt. Der ausgefallene Feststoff wird abgesaugt und mit ca. 3 ml Wasser gewaschen. Dann wird er mit Aceton vom Filter gewaschen und das Filtrat eingeengt. Man erhält 0.597 g Feststoff. Nach Reversed-Phase-Trennung mit Wasser/Acetonitril (Gradient) erhält man 93 mg (14 % d. Theorie) mit einer Reinheit von 98,6 % nach HPLC/MS.
¹H-NMR (400 MHz, d₆-DMSO): δ = 1.36 (s, 6H, 2xCH₃), 2.20 (s, 3H, Ar-CH₃), 7.29-7.31 (d, 1H, ArH), 7.35 (d,1H, ArH), 7.47-7.51 (m, 3H, ArH), 7.61 (br, 1H, NH), 7.63-7.67 (m, 2H, ArH), 10.83 (s, br, 1H, OH) ppm.

### Beispiel (X-1-a-1)

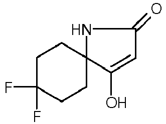

500 mg (1.9 mmol) der Verbindung gemäß Bsp. X-2-a-4 werden portionsweise innerhalb von 5 Minuten in ein siedendes 50%-iges Ethanol/Wassergemisch eingetragen. Man rührt unter Rückfluß bis die CO₂-Entwicklung beendet ist, rotiert ein und kristallisiert den Rückstand aus Ethanol um. Man erhält 275 mg eines farblosen Pulvers (69% d. Theorie).
¹H-NMR (400 MHz, CDCl₃): δ = 1.79-1.84 (m, 2H), 1.94 - 2.11 (2m, 4H), 2.20-2.33 (m, 2H), 3.13 (s, 2H, CO-CH₂-CO), 7.52 (br, 1H, NH) ppm.
¹H-NMR (400 MHz, CD₃CN): δ = 1.78-1.82 (m, 2H), 1.90 - 2.06 (2m, 4H), 2.12-2.18 (m, 2H), 3.03 (s, 2H, CO-CH₂-CO), 7.27 (br, 1H, NH) ppm.

### Beispiel (X-2-a-1)

### Verfahren B

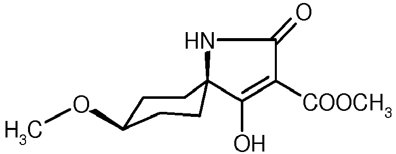

28.7 g (0.1 Mol) der Verbindung gemäß Bsp. XII-1 werden in 100 ml absoluten Methanol vorgelegt. Bei 20°C tropft man 19.5 ml Natriummmethylatlösung (30 %-ig in Methanol) zu und rührt 4 h bei 40°C nach. Das Lösungsmittel wird im Vakuum abgedampft , der Rückstand mit 50 ml Wasser aufgenommen und bei 0°C 110 ml 1 N Salzsäure zugetropft. Beim Eindampfen im Vakuum fällt das Produkt aus, wird anschließend in 50 ml Eiswasser suspendiert und abgesaugt. Ausbeute: 25 g (97 % d. Theorie) Fp. Zers.
¹H-NMR (400 MHz, d₆-DMSO): δ = 1.32-1.35 ("d", 2H), 1.39-1.49 (m, 2H), 1.65-1.73 (tm, 2H), 1.90-194 (dm, 2H), 3.09-3.16 (zm, 1H, CHOCH₃-cis), 3.24 (s, 3H, OCH₃), 3.59 (s, 3H, COOCH₃) ppm.
HPLC Retentionszeit 0.97 (Methode: Säule 50 x 4.6 mm Eclipse Plus C₁₈; 1.8 µm, Gradient 0.1% Phosphorsäure/Acetonitril; Fluss: 2 ml/min, 55 °C)

In Analogie zu Beispiel (X-2-a-1) und gemäß den allgemeinen Angaben zur Herstellung erhält man folgende Verbindungen der Formel (X-2-a):

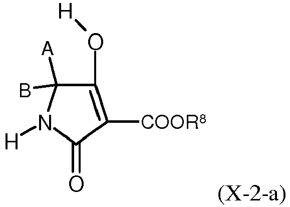

| **Bsp-Nr.** | **A** | **B** | **R⁸** | **Fp. °C; Analytik** * |
|---|---|---|---|---|
| X-2-a-2 | -(CH₂)₂-O-(CH₂)₂- | | CH₃ | 324; *¹ |
| X-2-a-3 | 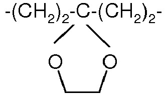 | | CH₃ | *² |
| X-2-a-4 | -(CH₂)₂-CF₂-(CH₂)₂- | | CH₃ | *³ |

*^{1 1}H-NMR (400 MHz, CD₃OD): δ = 1.43-1.47 (dd, 2H), 2.05-2.13 (tm, 2H), 3.66-3.72 (td, 2H, OCH₂), 3.80 (s, 3H, COOCH₃), 3.96-4.00 (d, m, 2H, OCH₂) ppm.
*^{2 1}H-NMR (400 MHz, d₆-DMSO): δ = 1.17-1.2 (d, 2H), 1.56-1.86 (m, 6H), 3.46 (s, 3H, CO₂CH₃), 3.84 (s, 4H, -O(CH₂)₂-O), 7.23 (br, 1H, NH) ppm.
*^{3 1}H-NMR (600 MHz, d₆-DMSO): δ = 1.52-1.54 (d, br, 2H), 1.91 (cm, br, 2H), 2.11-2.13 (2"d, br", 4H), 3.66 (s, 3H, CO₂CH₃), 8.85 (br, 1H, NH) ppm.

### Beispiel XII-1

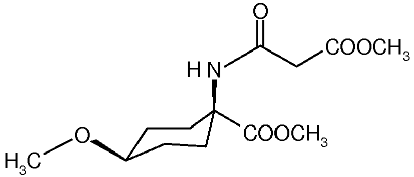

117.4 g (0.525 Mol) cis-1-Amino-4-methoxy-cyclohexancarbonsäure-methylester-hydrochlorid werden in 1000 ml absolutem Tetrahydrofuran (THF) vorgelegt, mit 153.3 ml (1.1 Mol) Triethylamin versetzt und bei 20°C 68.3 g (0.5 Mol) Malonsäuremethylesterchlorid in 30 ml absolutem THF zugetropft. Man rührt 4 h bei 40°C nach, gießt den Ansatz auf 1 l Wasser, extrahiert mit Methylenchlorid, trocknet die organische Phase und dampft im Vakuum ein. Der Rückstand (172 g) wird durch Säulenchromatographie an Kieselgel mit Methylenchlorid/Essigsäureethylester 2:1 als Laufmittel gereinigt.
Ausbeute: 85.6 g (59.6 % d. Theorie), Fp. 74°C
¹H-NMR (400 MHz, CD₃CN): δ = 1.34-1.44 (qm, 2H), 1.73-1.81 (tm, 2H), 1.85-195 (m, 2H), 2.06-2.12 (dm, 2H), 3.15-3.22 (zm, 1H, CHOCH₃-cis), 3.24 (s, 2H, CH₂COOCH₃), 3.28 (s, 3H, OCH₃), 3.60, 3.68 (2s, je 3 H, COOCH₃), 6.88 (s, br, 1 H, NH) ppm.

In Analogie zu Beispiel (XII-1) und gemäß den allgemeinen Angaben zur Herstellung erhält man folgende Verbindungen der Formel (XII):

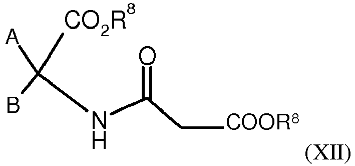

| **Bsp-Nr.** | **A** | **B** | **R⁸** | **Fp. °C; Analytik** * |
|---|---|---|---|---|
| XII-2 | -(CH₂)₂-O-(CH₂)₂- | | CH₃ | 62; *¹ |
| XII-3 | 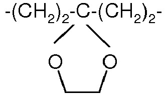 | | CH₃ | *² |
| XII-4 | -(CH₂)₂-CF₂-(CH₂)₂- | | CH₃ | *³ |

*^{1 1}H-NMR (400 MHz, CD₃CN): δ = 1.41-1.46 (dm, 1H), 1.86-2.08 (m, 3H), 3.25 (s, 2H, COCH2CO), 3.52-3.80 (m, 4H, OCH₂), 3.64, 3.68 (2s, je 3 H, COOMe), 7.06 (sbr, 1H, NH) ppm.
*^{2 1}H-NMR (400 MHz, CDCl₃): δ = 1.67-1.76 (m, 4H), 2.14-2.19 (m, 4H), 3.34 (s, 2H, CO-CH₂-CO), 3.72, 3.77 (2s, je 3H, CO₂CH₃), 3.96 (s, 4H,-O-(CH₂)₂-O), 7.67 (s, br, 1H, NH) ppm.
*^{3 1}H-NMR (400 MHz, d₆-DMSO): δ = 1.86-2.14 (m, 8H), 3.58 (s, 2H, COCH₂CO₂CH₃), 3.60, 3.63 (2s, je 3H, CO₂CH₃), 8.57 (s, br, 1H, NH) ppm.

### Beispiel X-1-b-1: 2,2-Dimethyl-5-oxo-2,5-dihydro-1H-pyrrol-3-ylacetat

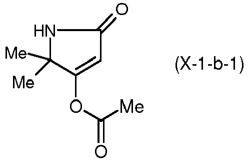

Zu einer Lösung von 6,36 g 5,5-Dimethylpyrrolidin-2,4-dion und 5,57 g Triethylamin in 50 ml Methylenchlorid tropft man bei 0 bis 5°C eine Lösung von 4,08 g Acetylchlorid in 20 Methylenchlorid. Man lässt innerhalb etwa einer Stunde auf Raumtemperatur kommen und rührt dann noch 24 Stunden. Anschließend wird das Reaktionsgemisch mit 50 ml Methylenchlorid verdünnt, zweimal mit je 50 ml Wasser, zweimal je 25 ml 5%iger Natronlauge und einmal 50 ml gesättigter wässriger NaCl-Lösung ausgeschüttelt. Nach Trocknen und Einengen erhält man 1,58 g der Titelverbindung in einer Reinheit n. HPLC von 97%.
LC/MS: m/e = 170 (MH⁺).
¹H-NMR (400 MHz, CDCl₃): δ = 1,33 (s, 6H), 2,23 (s, 3H), 5,91 (s, 1H), 7,05 (s,br, 1H) ppm.

### Beispiel X-1-c-1: 2,2-Dimethyl-5-oxo-2,5-dihydro-1H-pyrrol-3-yl-ethylcarbonat

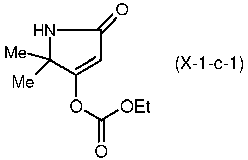

Zu einer Lösung von 6,36 g 5,5-Dimethylpyrrolidin-2,4-dion und 5,57 g Triethylamin in 50 ml Methylenchlorid tropft man bei 0 bis 5°C eine Lösung von 5,82 g Chlorameisensäure-ethylester in 20 Methylenchlorid. Man lässt innerhalb etwa einer Stunde auf Raumtemperatur kommen und rührt dann noch 24 Stunden. Anschließend wird das Reaktionsgemisch mit 50 ml Methylenchlorid verdünnt, zweimal mit je 50 ml Wasser, zweimal je 25 ml 5%iger Natronlauge und einmal 50 ml gesättigter wässriger NaCl-Lösung ausgeschüttelt. Nach Trocknen und Einengen erhält man 3,66 g der Titelverbindung in einer Reinheit n. HPLC von 98%.
LC/MS: m/e = 200 (MH⁺).
¹H-NMR (400 MHz, CDCl₃): δ = 1,31 - 1,35 (m, 9H), 4,25 - 4,30 (q, 2H), 5,88 (s, 1H), 7,29 (s,br, 1H) ppm.

### Beispiel XVIII-1:

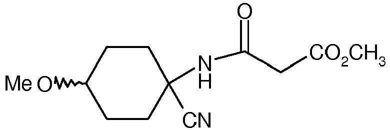

5.72 g (30 mmol) 1-Amino-4-methoxy-cyclohexancarbonsäurenitril hydrochlorid (cis/trans ca. 1:1) werden in 60 ml Tetrahydrofuran (THF) vorgelegt, mit 8.36 ml (60 mmol) Triethylamin und 10 mg Steglich-Base versetzt. Bei 0° - 10°C tropft man 4.1 g (30 mmol) Malonsäuremethylesterchlorid in 5 ml THF hinzu und rührt 4 h bei Raumtemperatur nach, saugt ab, wäscht mit THF nach und engt im Vakuum ein. Der Rückstand wird durch Flashsäulenchromatographie an Kieselgel mit Cyclohexan/Essigsäireethylester 2:1 gereinigt. Man erhält 4,96 g (65 % d. Theorie) eines cis/trans Isomerengemisches im Verhältnis von ca. 7:3.
¹H-NMR (400 MHz, d₆-DMSO): δ = 1,41-1.47 (m, 2H), 1.68-1.74 (m, 2H), 1.91-1.99 (m, 2H), 2.21-2.25 (m, 2H), 3.21, 3.24 (2s, trans/cis, zus. 3H, OCH₃), 3.22-3.27 (m, 1H, CHOCH₃), 3.32 (s, 2H, CH₂CO₂CH₃), 3.63 (s, 3H, CO₂CH₃), 8.56, 8.63 (2s, br, trans/cis, zus. 1H, NH) ppm.

### Beispiel XX-1:

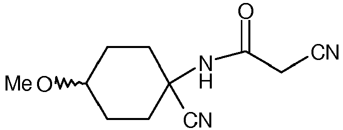

9.53 g (50 mmol) 1-Amino-4-methoxy-cyclohexancarbonsäurenitril x HCl (cis/trans Gemisch ca. 1:1) und 4.25 g (50 mmol) Cyanoessigsäure werden in 25 ml Pyridin vorgelegt. Anschließend tropft man ohne Kühlung 5.1 g (50 mmol) Acetonhydrid in 25 ml Pyridin zu und arbeitet nach der Zugabe sofort auf. Das Pyridin wird im Vakuum abgedampft, der Rückstand 2 x mit je 20 ml Toluol aufgenommen und wieder eingedampft. Anschließend wird Wasser zugegeben, mit Methylenchlorid extrahiert, getrocknet und eingedampft. Der Rückstand wird durch Flashchromatographie an Kieselgel mit Essigsäureetyhlester/Methanol Gradient 9:1 bis 4:1 vorgereinigt. Man erhält 6.86 g eines stark nach Essig riechenden Wachses, das aus 50 ml Essigsäureethylester umkristallisiert wird. Nach dem Absaugen erhält man 1.61 g (14.6 % d. Theorie) eines weißen Pulvers.
¹H-NMR (400 MHz, d₆-DMSO): δ = 1.37-1.46 (m, 2H), 1.67-1.72 (cm, 2H), 1.91-1.94 (m, 2H), 2.22-2.26 (m, 2H), 3.24 (s, 3H, OCH₃), 3.22-3.26 (m, 1H, CHOCH₃), 3,74 (s, 2H, CO-CH₂CN), 8.81 (s, br, 1H, NH) ppm.

## Patentansprüche

1. Verbindungen der Formel (X) 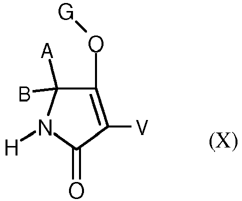 in welcher
A, B und das Kohlenstoffatom an das sie gebunden sind, für gesättigtes C₆-Cycloalkyl stehen, worin ein Ringglied durch Sauerstoff ersetzt ist oder
A, B und das Kohlenstoffatom an das sie gebunden sind, für gesättigtes C₆-Cycloalkyl, welches zweifach durch Fluor oder einfach durch Methoxy substituiert ist, oder
A, B und das Kohlenstoffatom, an das sie gebunden sind, für C₆-Cycloalkyl stehen, welches durch eine mit zwei nicht direkt benachbarte Sauerstoffatome enthaltende Alkylendiyl-Gruppe substituiert ist, die mit dem Kohlenstoffatom, an das sie gebunden ist, einen weiteren fünfgliedrigen Ring bildet,
G für Wasserstoff (a) oder 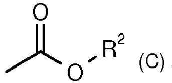 steht,
wobei R² für Ethyl steht,
V für Wasserstoff (X-1) oder COOR⁸ (X-2) steht,
wobei R⁸ für Methyl steht.

2. Verfahren zur Herstellung von Verbindungen der Formel (X) gemäß Anspruch 1, **dadurch gekennzeichnet, dass** man zum Erhalt von
B) Verbindungen der Formel (X-2-a) 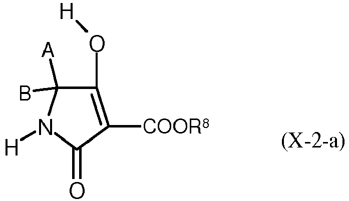 in welcher A, B und R⁸ die in Anspruch 1 angegebenen Bedeutungen haben, Verbindungen der Formel (XII) 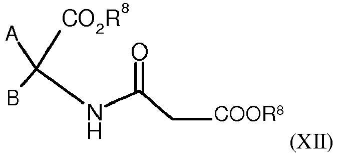 in welcher
A, B und R⁸ die in Anspruch 1 angegebenen Bedeutungen haben,
in Gegenwart eines Verdünnungsmittels und in Gegenwart einer Base intramolekular kondensiert,
(C) Verbindungen der Formel (X-1-a) 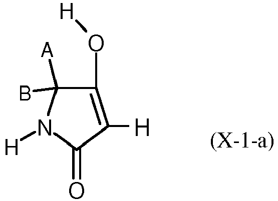 in welcher
A und B die in Anspruch 1 angegebenen Bedeutungen haben,
Verbindungen der Formel (X-2-a) 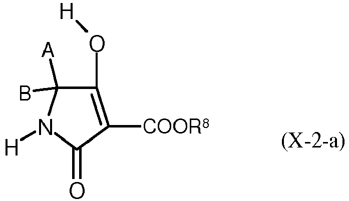 in welcher A, B und R⁸ die in Anspruch 1 angegebenen Bedeutungen haben, hydrolysiert und anschließend decarboxyliert,
(E) Verbindungen der oben gezeigten Formeln (X-1-c) oder (X-2-c), 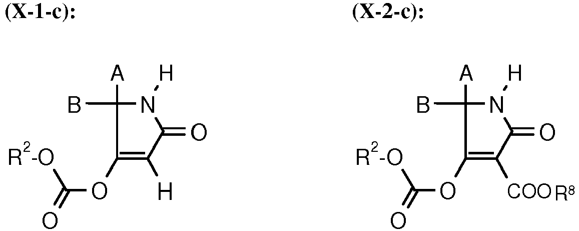 in welchen R², A, B und V die in Anspruch 1 angegebenen Bedeutungen haben, Verbindungen der oben gezeigten Formeln (X-1-a) und (X-2-a), in welchen A, B und V die in Anspruch 1 angegebenen Bedeutungen haben,
mit Chlorameisensäureestern der Formel (V)
R²-O-CO-Cl (V)
in welcher
R² die in Anspruch 1 angegebene Bedeutung hat,
gegebenenfalls in Gegenwart eines Verdünnungsmittels und gegebenenfalls in Gegenwart eines Säurebindemittels umsetzt.

## Claims

1. Compounds of the formula (X) 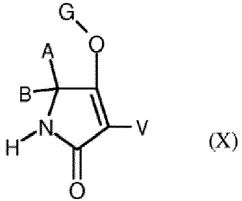 in which
A, B and the carbon atom to which they are attached are saturated C₆ cycloalkyl in which one ring member is replaced by oxygen or
A, B and the carbon atom to which they are attached are saturated C₆ cycloalkyl that is doubly substituted by fluorine or singly substituted by methoxy, or
A, B and the carbon atom to which they are attached are C₆ cycloalkyl that is substituted by an alkylenediyl group containing two oxygen atoms that are not directly adjacent, with said group forming a further five-membered ring with the carbon atom to which it is attached,
G is hydrogen (a) or 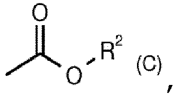
wherein R² is ethyl,
V is hydrogen (X-1) or COOR⁸ (X-2)
wherein R⁸ is methyl.

2. Method for preparing compounds of the formula (X) according to Claim 1, **characterized in that** in order to afford
B) compounds of the formula (X-2-a) 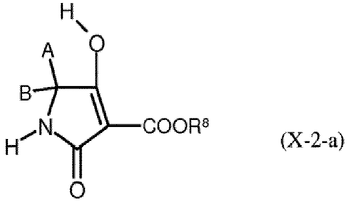 in which A, B and R⁸ are as defined in Claim 1,
compounds of the formula (XII) 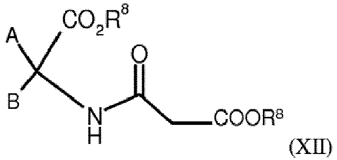 in which
A, B and R⁸ are as defined in Claim 1
are subjected to an intramolecular condensation in the presence of a diluent and in the presence of a base,
(C) compounds of the formula (X-1-a) 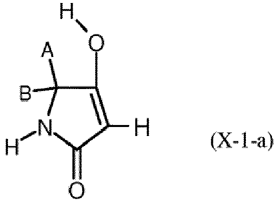 in which A and B are as defined in Claim 1,
compounds of the formula (X-2-a) 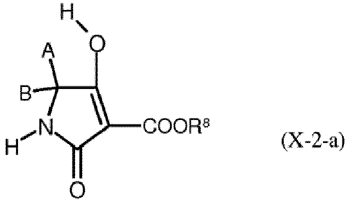 in which A, B and R⁸ are as defined in Claim 1,
are hydrolysed and subsequently decarboxylated.
(E) compounds of the formulas (X-1-c) or (X-2-c) shown above 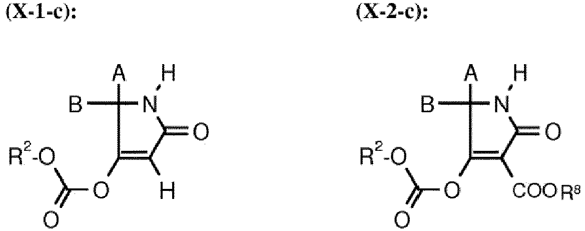 in which R², A, B and V are as defined in Claim 1, compounds of the formulas (X-1-a) and (X-2-a) shown above, in which A, B and V are as defined in Claim 1,
are reacted with chloroformic esters of the formula (V)
R²-O-CO-Cl (V)
in which
R² is as defined in Claim 1,
optionally in the presence of a diluent and optionally in the presence of an acid-binding agent.

## Revendications

1. Composés de formule (X) 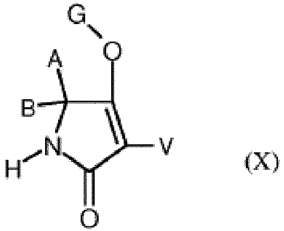 dans laquelle
A, B et l'atome de carbone auquel ils sont liés, représentent C₆-cycloalkyle saturé, dans lequel un élément de cycle est remplacé par oxygène ou
A, B et l'atome de carbone auquel ils sont liés, représentent C₆-cycloalkyle saturé, qui est substitué deux fois par fluor ou une fois par méthoxy, ou
A, B et l'atome de carbone auquel ils sont liés, représentent C₆-cycloalkyle, qui est substitué par un groupe alkylènediyle contenant deux atomes d'oxygène non directement adjacents, qui forme, avec l'atome de carbone auquel il est lié, un cycle supplémentaire à cinq chaînons,
G représente hydrogène (a) ou 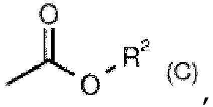 R2 représentant éthyle,
V représente hydrogène (X-1) ou COOR⁸ (X-2), R⁸ représentant méthyle.

2. Procédé pour la préparation de composés de formule (X) selon la revendication 1, **caractérisé en ce que** pour l'obtention de
B) composés de formule (X-2-a) 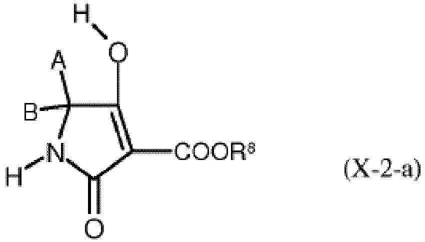 dans laquelle A, B et R⁸ possèdent les significations mentionnées dans la revendication 1,
on condense de manière intramoléculaire des composés de formule (XII) 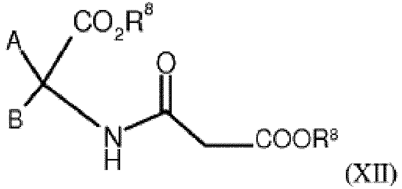 dans laquelle A, B et R⁸ possèdent les significations mentionnées dans la revendication 1,
en présence d'un agent de dilution et en présence d'une base,
(C) composés de formule (X-1-a) 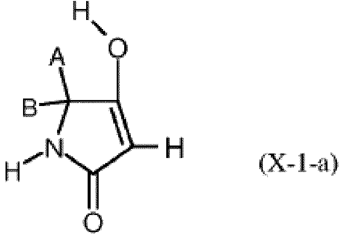 dans laquelle A et B possèdent les significations mentionnées dans la revendication 1,
on hydrolyse et ensuite on décarboxyle des composés de formule (X-2-a) 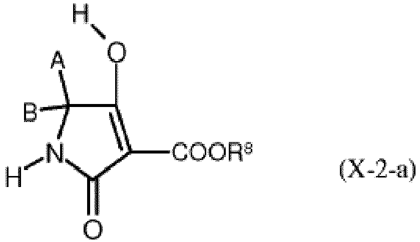 dans laquelle A, B et R⁸ possèdent les significations mentionnées dans la revendication 1,
(E) composés des formules représentées ci-dessus (X-1-c) ou (X-2-c), 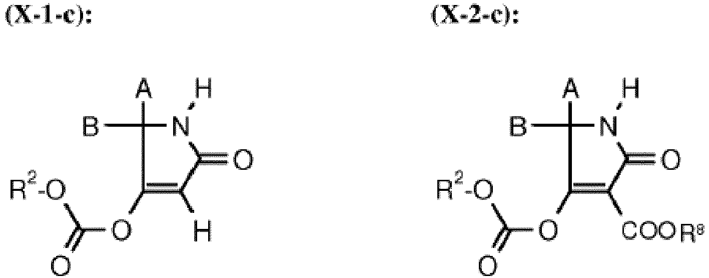 dans lesquelles R², A, B et V possèdent les significations mentionnées dans la revendication 1, on transforme des composés des formules représentées ci-dessus (X-1-a) et (X-2-a), dans lesquelles A, B et V possèdent les significations mentionnées dans la revendication 1,
avec des chlorures d'esters d'acide formique de formule (V)
R²-O-CO-Cl (V)
dans laquelle
R² possède la signification mentionnée dans la revendication 1,
éventuellement en présence d'un agent de dilution et éventuellement en présence d'un liant pour acide.
